# EUROPEAN PATENT APPLICATION

(11) **EP 4 385 516 A1**
(43) Date of publication of application: **19.06.2024**
(21) Application number: 21953150.6
(22) Date of filing: 12.08.2021
(51) Int. Cl.: A61K 38/08, A61K 38/17, A61K 38/05, A61K 8/64, A61K 47/42, A61P 17/02, A61Q 19/00

(54) **SYNTHETIC PEPTIDE, AND COSMETIC COMPOSITION OR PHARMACEUTICAL COMPOSITION AND APPLICATION THEREOF**

(71) Applicant: Shenzhen Winkey Technology Co., Ltd., Shenzhen, Guangdong 518000 (CN)
(72) Inventor: DING, Wenfeng, Shenzhen, Guangdong 518100 (CN); ZHAO, Wenhao, Shenzhen, Guangdong 518100 (CN); SUN, Xinlin, Shenzhen, Guangdong 518100 (CN); CHEN, Xue, Shenzhen, Guangdong 518100 (CN); PENG, Yan, Shenzhen, Guangdong 518100 (CN); GUAN, Fuyi, Shenzhen, Guangdong 518100 (CN)
(74) Representative: Bandpay & Greuter
(86) International application number: PCT/CN2021/112345
(87) International publication number: WO 2023/015533

(57) **Abstract**

A peptide as represented by general formula (I), a stereoisomer thereof, a mixture of the stereoisomer, or a cosmetically acceptable salt or pharmaceutically acceptable salt thereof, or a cosmetic composition or pharmaceutical composition thereof, and an application thereof for treatment or care of a disorder, an illness, or a disease of the skin or mucosa.

## Description

### Technical Field

The present invention relates to peptides capable of promoting the proliferation of fibroblasts in the skin or mucosa, as well as cosmetic or pharmaceutical compositions containing these peptides and their use in the treatment or care of the skin or mucosa.

### Background Technique

The skin is the largest organ of the human body. It covers the body surface and plays an important barrier role for the body. It can resist mechanical damage, buffer external shocks, avoid damage to tissues and organs in the body, prevent the invasion of harmful substances from the outside, and resist oxidation and UV damage. It can also prevent the loss of various nutrients, water, and electrolytes in the body, and plays an important role in maintaining the stability of the internal environment of the human body. Skin consists of epidermis, dermis, and subcutaneous tissue. The outermost layer is the epidermis, which is mainly composed of keratinocytes, melanocytes, and Langerhans cells. The dermis is located below the epidermis and contains dermal cells, collagen fibers, elastic fibers, matrix, hair, sebaceous glands, sweat glands, blood vessels, etc. The dermis is tightly connected to the epidermis by a basement membrane.

The most dominant cells in the dermis are fibroblasts. Fibroblasts are large in size and diverse in shape, mostly protruding spindle-shaped or star-shaped flat structures. The cytoplasm contains a large amount of developed rough endoplasmic reticulum, Golgi apparatus, and free ribosomes, so it has a strong ability to synthesize secretory proteins and in turn can secrete extracellular matrix components such as procollagen, fibronectin, and collagenase. Fibroblasts can use amino acids such as proline and lysine to synthesize proalpha polypeptide chains on the ribosomes of the rough endoplasmic reticulum, and then the produced polypeptide chains are transported to the Golgi apparatus to form procollagen molecules, which are then released extracellularly by exocytosis. Under the catalysis of procollagen peptidase, the tail segment of the pre-alpha polypeptide chain of the procollagen molecule is removed to become the tropocollagen molecule. Tropocollagen molecules are arranged in parallel rows and combined into collagen fibrils with periodic horizontal striations. Collagen fibrils combine with each other to form collagen fibers. In addition to producing collagen fibers, fibroblasts can also produce elastic fibers. The main component of collagen fibers is type I collagen, which has strong tensile strength and gives skin toughness. Elastic fibers, which formed by aggregation of elastin and fibroin, can give the skin its elasticity. Collagen fibers, elastic fibers, and extracellular matrix filled between fibers and cells form a support for the skin.

When aging, the skin's morphology, physiology, and its mechanical properties undergo significant changes. During the natural aging process of the skin, the function of fibroblasts declines, collagen fibers and elastic fibers are damaged and broken, and the balance between the production and degradation of important biomolecules such as collagen in the skin is biased towards the degradation process with age, resulting in dermis thinning and tissue destruction progressively, and in turn causing relaxation of the dermis followed by the formation of wrinkles. Prolonged exposure to sunlight or environmental pollutants can accelerate the skin aging process. Ultraviolet rays can inhibit the synthesis of collagen and fibronectin in fibroblasts, cause high expression of matrix metalloproteinases (MMPs), cause serious damage to collagen in the dermis, destruct dermal integrity, and decrease skin strength and elasticity, resulting in the acceleration of skin aging.

Therefore, by promoting the proliferation of fibroblasts, enhancing their activity, and increasing the synthesis and secretion of collagen, elastin, glycosaminoglycans and other components, it is possible to improve the loss of skin mechanical properties such as flexibility, elasticity and firmness with aging or exposure to sunlight and/or exposure to environmental pollutants, so as to repair damaged skin and reduce wrinkles.

As the first line of defense of the human body, the skin is vulnerable to external damage, thus losing its barrier function and weakening the resistance. Non-pathogenic bacteria or opportunistic pathogenic bacteria will invade the skin, induce infection, and may even have a major impact on physical health. Therefore, accelerating the repair of skin wounds is of great significance in treating diseases with refractory wounds and postoperative repair.

Wound repair is a precise, complex, time-phased dynamic process involving network regulation of various cells such as cell proliferation, migration, degradation of extracellular matrix, angiogenesis, and epithelial tissue remodeling. Fibroblasts are the main cells in wound repair. Mature fibroblasts are in a quiescent state, and their cell body volume is reduced, showing a long spindle shape, rough endoplasmic reticulum and Golgi apparatus are reduced, and mature fibroblasts are called fibrocytes. Fibroblasts and fibrocytes can transform into each other under certain conditions. Under the stimulation of trauma and other factors, some fibrocytes retransform into immature fibroblasts, and their synthetic and secretory functions are restored accordingly, stimulating the generation of new extracellular matrix, and a large amount of extracellular matrix is combined with new capillaries to form granulation tissue to fill the defect organize. During the repair process, the proliferation and migration of fibroblasts are crucial to the formation of granulation tissue and the repair of wounds. Studies have shown that fibroblasts are mainly involved in the proliferation phase and tissue remodeling phase of wound repair. During the proliferative phase, fibroblasts proliferate significantly, migrate and form granulation tissue, which mainly play the role of resisting infection and filling wounds. During the tissue remodeling period, myofibroblasts, endothelial cells and macrophages differentiated from fibroblasts undergo apoptosis, and finally the dermal tissue is filled with extracellular matrix proteins to achieve the purpose of wound repair and maintenance of the integrity of skin structure and function.

To sum up, fibroblasts play an important role in the process of skin aging and wound repair. It is of great significance to develop a product that can promote the proliferation of fibroblasts, enhance the activity of fibroblasts, increase the synthesis and secretion of collagen, elastin, glycosaminoglycans and other ingredients to compensate for the loss of extracellular matrix such as collagen and elastin with aging or exposure to sunlight and/or exposure to environmental pollutants, delay aging, fill the vacancy of necrotic tissue area after skin injury, and promote wound repair.

### Contents of the Invention

The inventors of the present invention have surprisingly found that the proliferation and activity of fibroblasts can be regulated by certain synthetic peptides through tremendous experimental studies. The present invention aims to provide a peptide capable of promoting proliferation of fibroblasts in skin or mucosa. These peptides and cosmetic compositions or pharmaceutical compositions containing these peptides can improve, prevent, treat, or care of conditions, disorders, or diseases of the skin or mucosa by promoting the proliferation of fibroblasts; by improving the skin barrier function; by treating, preventing, or repairing skin aging or photoaging; or by re-epithelializing and/or healing of skin or mucosa.

In view of this, the present invention provides a peptide represented by general formula (I), or its stereoisomer, or a mixture of stereoisomers, or its cosmetically acceptable salt, or its pharmaceutically acceptable salt, characterized by:

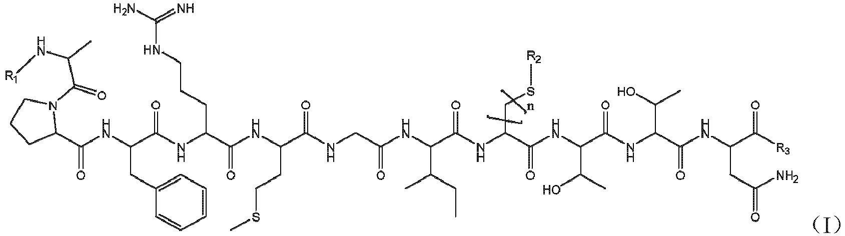

In formula (I),
R₂ is selected from: H, alkyl, alkenylalkyl, carboxyalkyl, esterylalkyl, alkylmercapto, carboxyalkylmercapto, or

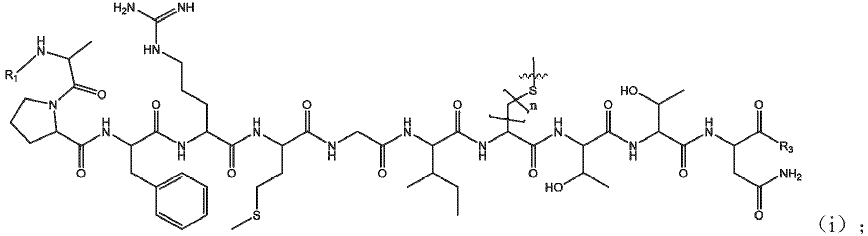

In formula (I) and formula (i),
n is selected from: 1 or 2;
R₁ is selected from: H or R₄-CO-, wherein R₄ is selected from: substituted or unsubstituted alkyl, substituted or unsubstituted alkenyl;
R₃ is selected from: -NR₅R₆ or -OR₅, wherein each R₅ and R₆ are independently selected from: H, substituted or unsubstituted alkyl, substituted or unsubstituted alkenyl;

The groups R₁ and R₃ are bound to the amino-terminus (N-terminus) and carboxy-terminus (C-terminus) of these peptide sequences, respectively.

The alkyl in the "alkyl", "alkenylalkyl", "carboxyalkyl", "esterylalkyl", "alkylmercapto", "carboxyalkylmercapto" refers to a saturated aliphatic straight-chain or branched alkyl group having 1-24 carbon atoms (optionally having 1-16 carbon atoms; optionally having 1-14 carbon atoms; optionally having 1-12 carbon atoms; optionally having 1, 2, 3, 4, 5, or 6 carbon atoms); optionally is selected from: methyl, ethyl, isopropyl, isobutyl, tert-butyl, pentyl, hexyl, heptyl, octyl, decyl, dodecyl, tetradecyl, hexadecyl, octadecyl, 2-ethylhexyl, 2-methylbutyl, or 5-methylhexyl;

The alkenyl in the "alkenyl" and "alkenylalkyl" refers to a straight-chain or branched alkenyl group having 2-24 carbon atoms (optionally having 2-16 carbon atoms; optionally having 2-14 carbon atoms; optionally having 2-12 carbon atoms, optionally having 2, 3, 4, 5, or 6 carbon atoms); having one or more carbon-carbon double bonds, optionally having 1, 2 or 3 conjugated or non-conjugated carbon-carbon double bonds; said "alkenyl" is bonded to the rest of the molecule through a single bond; optionally selected from: vinyl, oleyl, or linoleyl;

The esteryl in the "esterylalkyl" is an R(C=O)OR' group, wherein R and R' are each independently a single bond, an alkyl group or an alkylene group;

As is understood in the art, some degree of substitution may exist in the groups described above. Substitution may therefore exist in any of the groups in the invention. When refer to substituted groups in the groups of the present invention, it is indicated that the specified group may be substituted with one or more substituents in one or more available positions, preferably in 1, 2, or 3 positions, more preferably in 1 or 2 positions, still more preferably in one position. Such substituents include but not limited to, for example: C₁-C₄ alkyl; hydroxy; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthiol; C₆-C₃₀ aryloxy such as phenoxy; -NR_{b}(C==NR_{b})NR_{b}R_{c}, wherein R_{b} and R_{c} are independently selected from: H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, three to ten membered heterocyclic group, or protecting group of amino group. Optionally, in formula (I), when n is 2, R₂ is selected from: H, alkyl, alkenylalkyl, carboxyalkyl, esterylalkyl, alkylmercapto or carboxyalkylmercapto.

Optionally, R₁ is selected from: H, acetyl, tert-butyryl, hexanoyl, 2-methylhexanoyl, capryl, decanoyl, lauroyl, myristoyl, palmitoyl, stearoyl, oleoyl acyl or linoleoyl.

Optionally, R₁ is selected from H, acetyl, lauroyl, myristoyl or palmitoyl.

Optionally, R₁ is H, acetyl or palmitoyl.

Optionally, R₂ is selected from: H, -CH₃, -CH₂CH₃, -CH₂CH=CH₂, -CH₂COOH, - CH₂COOC(CH₃)₃, -S-CH₂CH₃, -S-(CH₂)₅CH₃, -S-CH₂COOH or

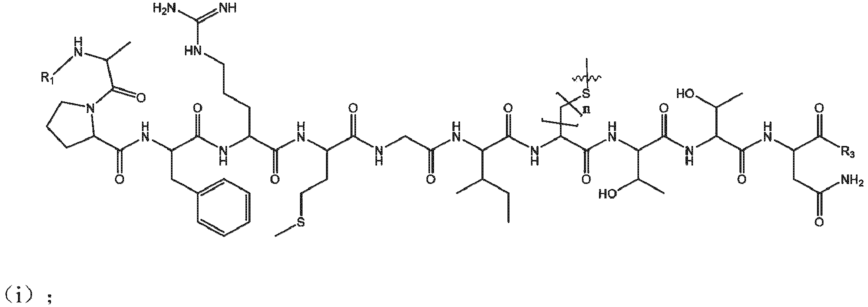

Optionally, when n is 2, R₂ is selected from: H, -CH₃, -CH₂CH₃, -CH₂CH=CH₂, -CH₂COOH, - CH₂COOC(CH₃)₃, -S-CH₂CH₃, -S-(CH₂)₅CH₃ or -S-CH₂COOH.

Optionally, R₅ and R₆ are independently selected from: H, methyl, ethyl, hexyl, dodecyl or hexadecyl;

Optionally, R₅ is H and R₆ is selected from: H, methyl, ethyl, hexyl, dodecyl and hexadecyl; Optionally, R₃ is -OH or -NH₂.

The peptide represented by formula (I), or its stereoisomer, or a mixture of stereoisomers, or its cosmetically acceptable salt, or its pharmaceutically acceptable salt, is selected from the following peptides (1)-(46): 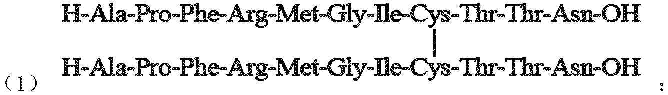
(2) H-Ala-Pro-Phe-Arg-Met-Gly-Ile-Met-Thr-Thr-Asn-OH; 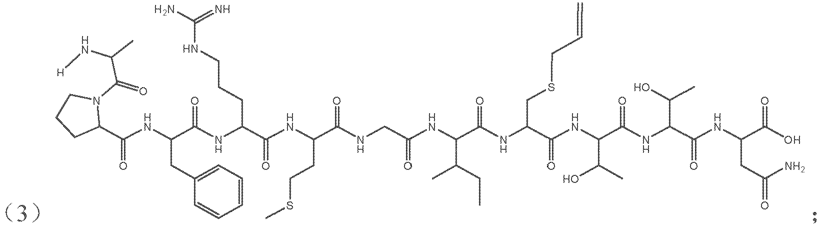 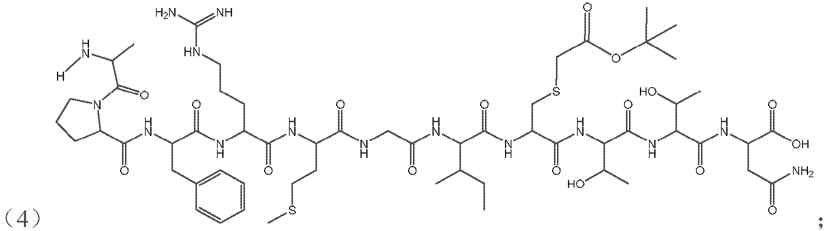 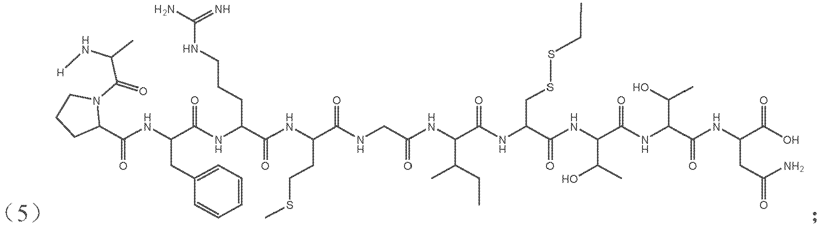 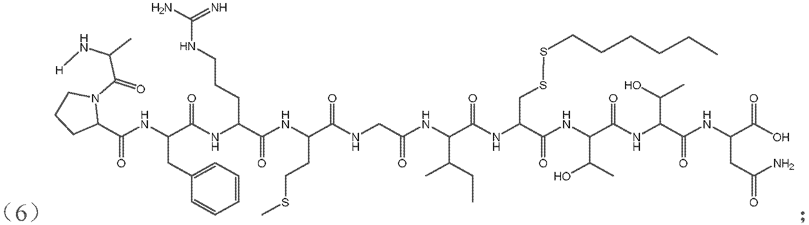 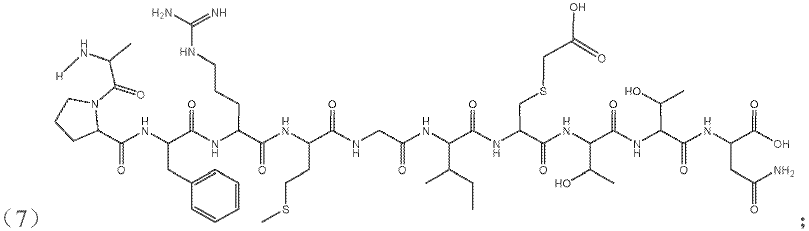
(8) Palm-Ala-Pro-Phe-Arg-Met-Gly-Ile-Cys- Thr- Thr-Asn-OH;
(9) Palm-Ala-Pro-Phe-Arg-Met-Gly-Ile-Cys-Thr-Thr-Asn-NH₂;
(10) Ac-Ala-Pro-Phe-Arg-Met-Gly-Ile-Cys- Thr- Thr-Asn-OH;
(11) Ac-Ala-Pro-Phe-Arg-Met-Gly-Ile-Cys-Thr-Thr-Asn-NBh; 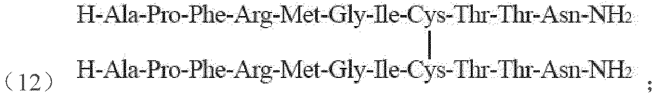 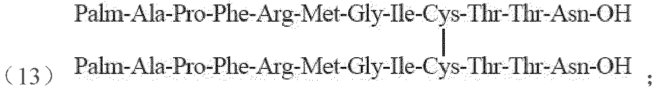 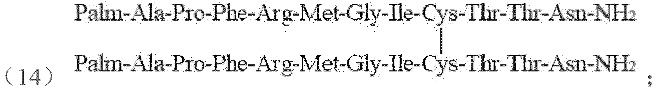 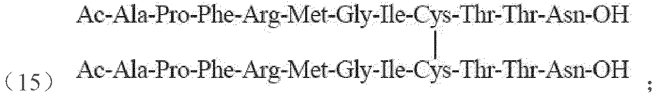 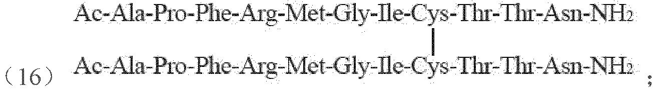 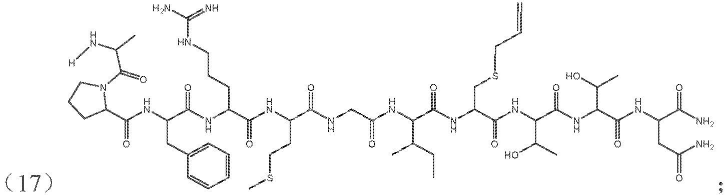 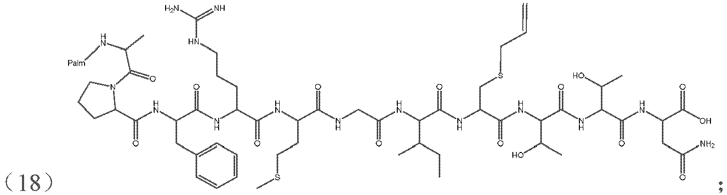 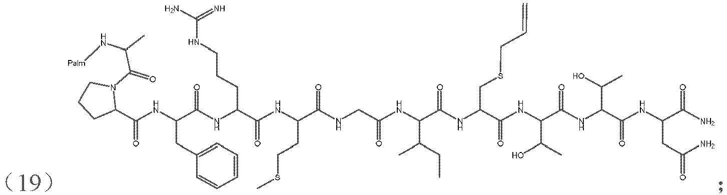 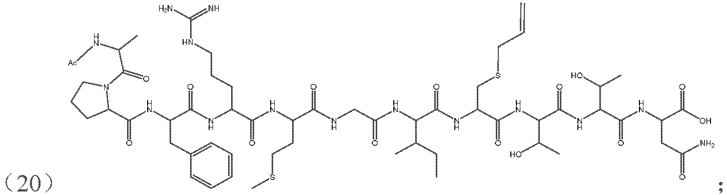 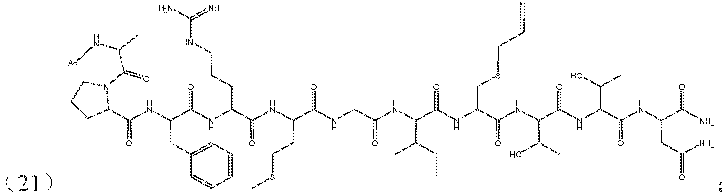 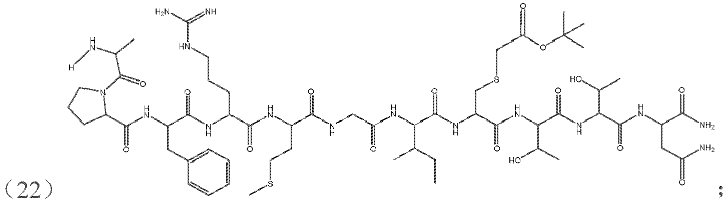 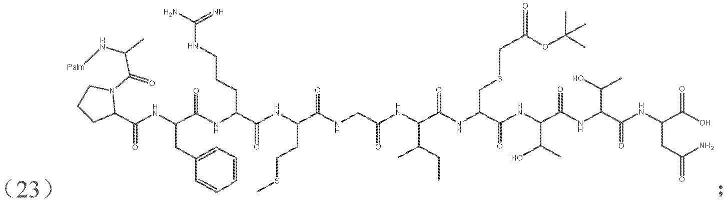 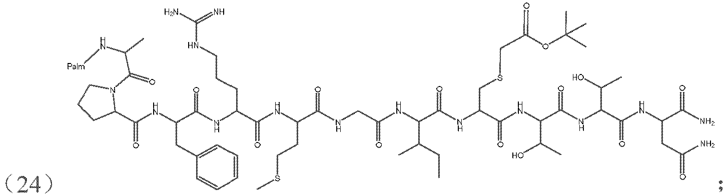 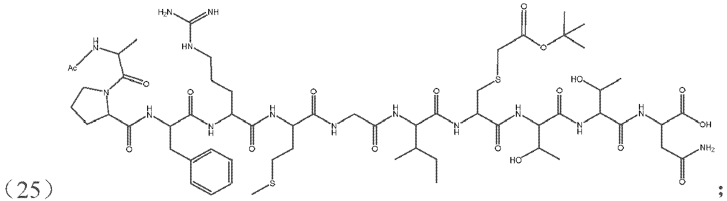 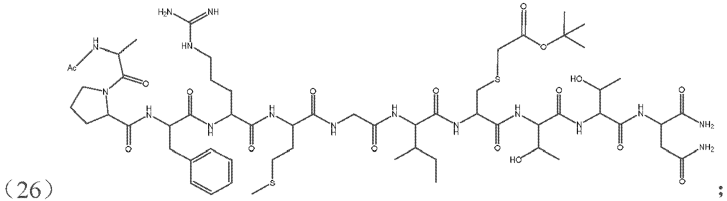 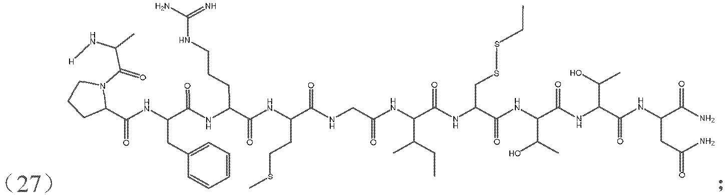 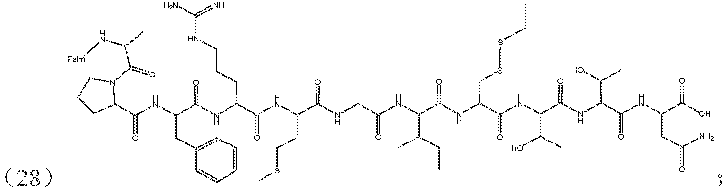 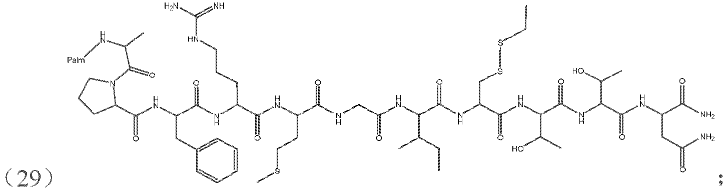 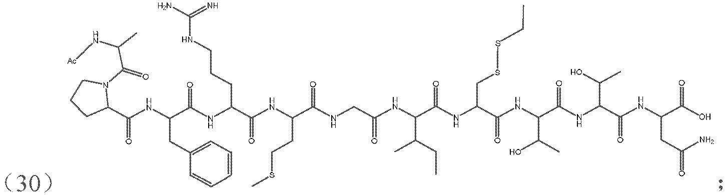 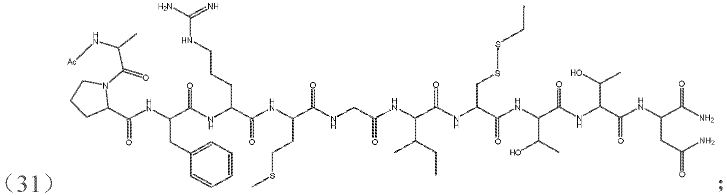 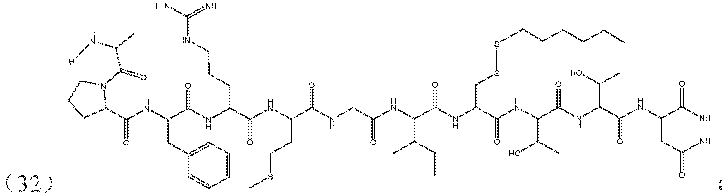 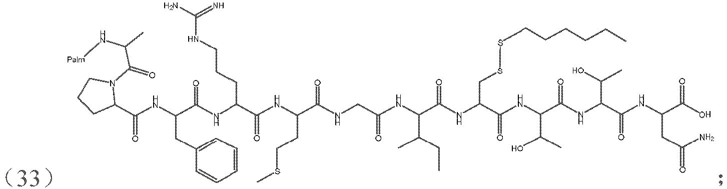 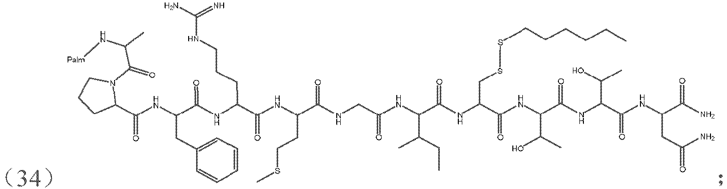 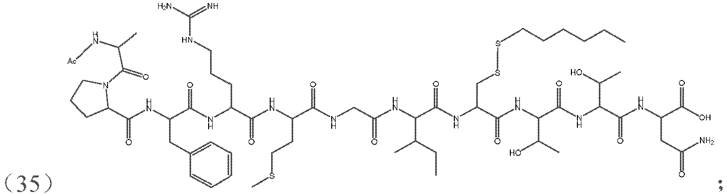 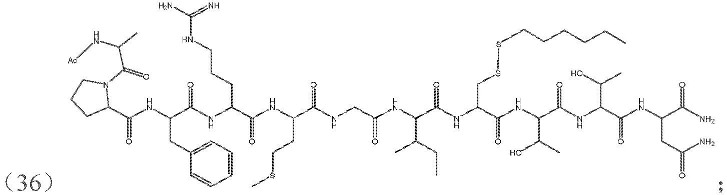 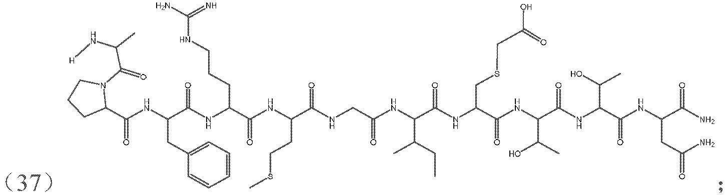 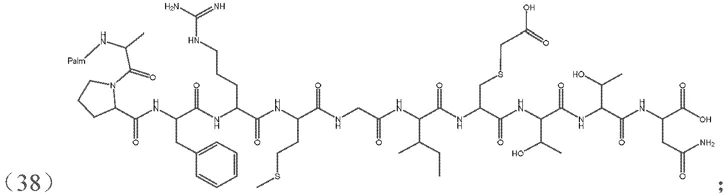 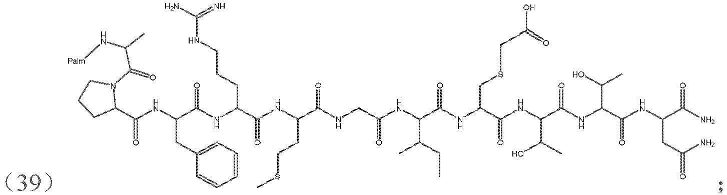 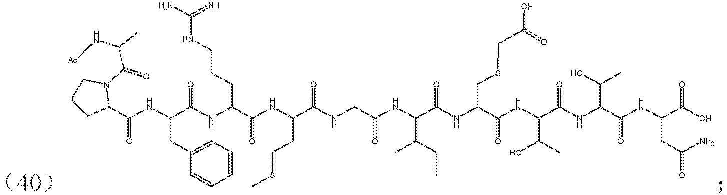 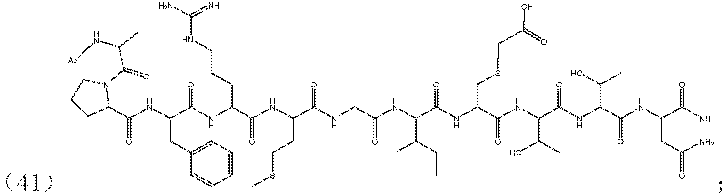
(42) H-Ala-Pro-Phe-Arg-Met-Gly-Ile-Met-Thr-Thr-Asn-NH₂;
(43) Palm-Ala-Pro-Phe-Arg-Met-Gly-Ile-Met-Thr-Thr-Asn-OH;
(44) Palm-Ala-Pro-Phe-Arg-Met-Gly-Ile-Met-Thr-Thr-Asn-NH₂;
(45) Ac-Ala-Pro-Phe-Arg-Met-Gly-Ile-Met-Thr-Thr-Asn-OH;
(46) Ac-Ala-Pro-Phe-Arg-Met-Gly-Ile-Met-Thr-Thr-Asn-NH₂.

The peptide represented by formula (I) of the present invention may exist as a stereoisomer or a mixture of stereoisomers; for example, these amino acid residues contained therein may have the configuration of L-, D-, or independently of each other racemic. Thus, it is possible to obtain isomeric mixtures as well as racemic or diastereomeric mixtures, or pure diastereomers or enantiomers, depending on the number of asymmetric carbons and the existence of isomers or isomeric mixtures. Preferred structures of the peptides represented by formula (I) of the present invention are pure isomers, ie, enantiomers or diastereomers.

For example, when -Phe- is mentioned in the present invention, it should be understood that - Phe- is selected from -L-Phe-, -D-Phe-, or a mixture of both, and is racemic or non-racemic. The preparation methods described in this document enable a person skilled in the art to obtain each stereoisomer of the peptides of the invention by selecting the amino acid with the correct configuration.

The term "cosmetically acceptable salt or pharmaceutically acceptable salt" refers to a salt approved for use in animals, and more specifically in humans, including metal salts of peptides represented by formula (I), and the metals include, but are not limited to: lithium, sodium, potassium, calcium, magnesium, manganese, copper, zinc or aluminum, etc.; including salts formed by the peptide represented by formula (I) and an organic base, and the organic base includes, but not limited to: ethylamine, diethylamine, ethylenediamine, ethanolamine, diethanolamine, arginine, lysine, histidine or piperazine, etc.; including salts formed by the peptide represented by formula (I) and inorganic acids or organic acids including, the organic acid includes, but not limited to: acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid (pamoate) or gluconic acid, etc.; the inorganic acid includes, but not limited to: hydrochloric acid, sulfuric acid, boric acid or carbonic acid.

The synthesis of the peptide, or its stereoisomer, or its cosmetically acceptable salt, or pharmaceutically acceptable salt of formula (I) of the present invention can be carried out according to conventional methods known in the prior art, such as using Solid Methods for Phase Peptide Synthesis [Stewart J.M. and Young J.D., "Solid Phase Peptide Synthesis", Second Edition, (1984), Pierce Chemical Co. Chemical Company, Rockford, Illinois; Bodanzsky M., Bodanzsky A. "The Practice of Peptide Synthesis", (1984), Springer Verlag, New York; Lloyd-Williams P., Albericio F., Giralt E. "Peptides and Chemical Approaches to the Synthesis of Peptides and Proteins", (1997), CRC, Boca Raton, FL, USA], Synthesis In Solution, Solid Phase Synthesis, And Synthesis In Solution Combination Of Methods Or Enzymatic Synthesis [Kullmann W. (Kullmann W.), "Proteases as catalysts for enzymic syntheses of opioid peptides" (1980), Biochemical Journal (J. Biol. Chem.), 255, 8234-8238]. Peptides can also be prepared by biotechnological methods aiming at producing the desired sequence, or by controlled hydrolysis of proteins with animal, fungal, or preferably plant origin, which releases peptide fragments containing at least the desired sequence.

For example, a method for obtaining peptides of general formula (I) comprises the following steps:
- Coupling an amino acid with a protected N-terminus and a free C-terminus to an amino acid with a free N-terminus and a protected C-terminus or bound to a solid support;
- Eliminating the group protecting the N-terminus;
- Repeating the coupling sequence and eliminating the group protecting the N-terminus until the desired peptide sequence is obtained;
- Eliminating the group protecting the C-terminus or cleavage from the solid support.

Preferably, the C-terminus is bound to a solid support and the method is carried out on a solid phase, comprising coupling an amino acid having a protected N-terminus and a free C-terminus with an amino acid having a free N-terminus and a C-terminus bound to the polymer carrier; eliminating the group protecting the N-terminus; and repeating this procedure as required to thereby obtain a peptide of the desired length, followed by cleavage of the peptide from the original polymer carrier to produce the synthetic peptides.

The functional groups of the side chains of the amino acids remain fully protected with temporary or permanent protecting groups throughout the synthesis and can be deprotected simultaneously or orthogonally with the process of cleavage of the peptide from the polymeric support. Alternatively, solid phase synthesis can be performed by a convergent strategy in which dipeptides or tripeptides are coupled to a polymeric support or to dipeptides or amino acids previously bound to a polymeric support. Convergent synthesis strategies are well known to those skilled in the art and are described in Lloyd-Williams P., Albericio F. and Giralt E. "Convergent Solid Phase Peptide Synthesis", (1993), Tetrahedron 49:11065- 11133.

The method may comprise the additional steps: deprotecting the N-terminus and C-terminus and/or cleaving the peptide from the polymer support in a non-determined order using standard conditions and methods known in the art, and the resulted terminal functional groups can be modified subsequently. Optional modifications of the N-terminus and C-terminus can be carried out on the peptide of general formula (I) bound to a polymeric support, or can be carried out after the peptide has been cleaved from the polymeric support.

The peptides of the present invention can be administered in the form of compositions containing thereof by any means that cause contact between these peptides and the site of action in mammals (preferably, the site of action in humans) to promote fibroblasts proliferation and increase collagen production.

For this reason, another aspect of the present invention provides a cosmetic composition or a pharmaceutical composition, comprising an effective amount of the above-mentioned peptide of formula (I), or its stereoisomer, or a mixture of stereoisomers, or a cosmetically acceptable salt thereof, or a pharmaceutically acceptable salt thereof, and at least one excipient or optionally a cosmetically or pharmaceutically acceptable adjuvant. These compositions can be prepared by conventional methods known to those skilled in the art ["Harry's Cosmetology", Seventh Edition, (1982), Wilkinson J.B. (Wilkinson J.B.), Moore R.J. (Moore R.J., Longman House, Essex, UK]. Optionally, the adjuvant is selected from: a collagen synthesis stimulator, agents that regulates the synthesis of PGC-1α, agents that regulates the activity of PPARγ, agents that increases or decreases the triglyceride content of adipocytes, agents that stimulate or delays the adipocyte differentiation, lipolytic agents or lipolysis stimulating agents, lipotropic agents, adipogenic agents, inhibitors of acetylcholine receptor aggregation, agents that inhibit muscle contraction, anticholinergic agents, elastase inhibitors, matrix metalloproteinases inhibitors, melanin synthesis stimulators or inhibitors, whitening or depigmenting agents, hyperpigmentation agents, self-tanning agents, antiaging agents, NO-synthase inhibitors, 5α-reductase inhibitors, lysyl hydroxylation enzyme and/or prolyl hydroxylase inhibitors, antioxidants, free radical scavengers and/or anti-air pollution agents, active carbonyl species scavengers, anti-glycation agents, antihistamines, antiviral agents, antiparasitic agents, emulsifiers, emollients, organic solvents, liquid propellants, skin conditioners, humectants, moisture retaining substances, alpha hydroxy acids, beta hydroxy acids, moisturizers, epidermal hydrolytic enzymes, vitamins, amino acids, proteins, pigments or colorants, dyes, biopolymers, gelling polymers, thickeners, surfactants, softeners, adhesives, preservatives, anti-wrinkle agents, agents that reduce or treat bags under the eyes, callus remover, exfoliating agents, keratolytic agents, antimicrobial agents, antifungal agents, fungicides, bactericides, bacteriostatic agents, agents that stimulate the synthesis of dermal or epidermal macromolecules and/or are capable of inhibiting or preventing their degradation, agents that stimulate elastin synthesis, agents that stimulate decorin synthesis, agents that stimulate laminin synthesis, agents that stimulate defensin synthesis, agents that stimulate chaperone protein synthesis, agents that stimulate cAMP synthesis, heat shock proteins, agents that stimulate the synthesis of HSP70, agents that stimulate the synthesis of heat shock proteins, agents that stimulate the synthesis of hyaluronic acid, agents that stimulate the synthesis of fibronectin, agents that stimulate the synthesis of sirtuin, agents that stimulate the synthesis of lipids and stratum corneum components, ceramide, fatty acid, agents for inhibiting collagen degradation, agents for inhibiting elastin degradation, agents for inhibiting serine protease, agents for stimulating fibroblast proliferation, agents for stimulating keratinocyte proliferation, agents for stimulating adipocyte proliferation, agents that stimulate melanocyte proliferation, agents that stimulate keratinocyte differentiation, agents that inhibit acetylcholinesterase, skin relaxants, agents that stimulate glycosaminoglycan synthesis, antihyperkeratosis agents, acne dissolving agents, antipsoriatic agents, anti-dermatitis agents, anti-eczema agents, DNA repair agents, DNA protection agents, stabilizers, antipruritic agents, agents for the treatment and/or care of sensitive skin, curing agents, firming agents, restructuring agents, anti-stretch mark agents, adhesives, agents that regulate sebum production, antiperspirants, agents that stimulate healing, agents that assist healing, agents that stimulate re-epithelialization, agents that assist re-epithelialization, cytokines, growth factors, sedatives, anti-inflammatory agents, anesthetic agents, agents acting on capillary circulation and/or microcirculation, agents stimulating angiogenesis, agents inhibiting vascular permeability, venous tense agents, agents acting on cell metabolism, agents for improving dermal-epidermal junction agents, agents that induce hair growth, agents that inhibit or delay hair growth, fragrances, chelating agents, plant extracts, essential oils, marine extracts, agents derived from biological fermentation processes, inorganic salts, cell extracts, sunscreens, and organic or inorganic light protective agents or mixtures thereof that effective against A and/or B UV rays, provided that they are physically and chemically compatible with the components of the composition, and in particular with the peptides of general formula (I) contained in the composition of the invention. Furthermore, the nature of these additional ingredients should not unacceptably alter the benefits of the peptides of the invention. The nature of these additional ingredients may be synthetic or natural such as plant extracts, or from a biotechnological process, or from a combination of a synthetic and a biotechnological process. Additional examples can be found in "CTFA International Cosmetic Ingredient Dictionary & Handbook", 12th Edition (2008). In the context of the present invention, a biotechnological method is understood as any method for producing the active ingredient, or a part of the active ingredient, in an organism, or in a part of an organism.

Alternatively, the above-mentioned cosmetic composition or pharmaceutical composition of the present invention may contain a cosmetically or pharmaceutically effective amount of at least one synthetic compound, plant extract, or product obtained by biotechnological methods, which is a collagen synthesis stimulating agent such as, without limitation, ascorbic acid and its derivatives such as ascorbyl palmitate, magnesium ascorbyl phosphate, sodium ascorbyl phosphate and ascorbyl α- and β-glucosides, retinol and retinol derivatives such as retinoic acid, retinol xanthal, retinyl acetate or retinyl palmitate, vegetable extracts such as aloe vera and centella asiatica, carnitine, carnosine, creatine, asiatic acid, madecassic acid, centella asiatica grass glycosides, asiatica saponin, centella asiatica extract, niacinamide, asaxanthin, dextran, soybean extracts and soybean isoflavones such as genistein and daidzein, rutin, chrysin, morin, areca nut alkaloids, forskolin, betulinic acid, plantago extract, TGF-beta, ginkgo biloba extract, glutamine, glycolic acid, [INCI: Palmitoyl Pentapeptide- 4], Matrixyl [INCI: palmitoyl tetrapeptide-7, palmitoyl oligopeptide] or [INCI: C12-15 alkyl benzoate, tribehenin, ceramide 2, PEG10 rapeseed sterol, Palmitoyl Oligopeptide] sold by Sederma/Croda, -Coll [INCI: Palmitoyl Tripeptide-5], [INCI: Locust Bean (Ceratonia Siliqua) Gum] sold by Pentapharm/DSM, or BeauActiveTM MTP [INCI: Hydrolyzed Milk Protein], ThalassineTM [INCI: Algae (Algae) Extract] sold by Biotechmarine, EquiStatTM [INCI: Apple Fruit Extract, Wild Soybean Seed Extract] or Juvenesce [INCI : Ethoxydiglycol and Caprylic Triglycerides, Retinol, Ursolic Acid, Vitamin K1, Ilomastat] sold by Coletica/Engelhard/BASF, SMS Anti-[INCI: Low Purified from Annona Squamosa] Polypeptide] or Papilactyl [INCI: Water, Cyperus Esculentus tuber extract] sold by Silab, ChroNOIineTM [INCI: Glycerol, Water (Aqua), Dextran, Hexanoyl Tetra Peptide-3 (Caprooyl Tetrapeptide-3)], [INCI: Tripeptide-1] or 4 [INCI: Water, Dextran, Acetyl Tetrapeptide-2] sold by Atrium Innovations/Unipex Group, [INCI: Protooligomeric peptide, composed of Lysine and L-Arginine] or [INCI: Arginine/Lysine Polypeptide] sold by Solabia BioLyse T.A., In-Tense [INCI: Caprylic/Capric Triglyceride, Golden Button (Spilanthes Acmella) Flower Extract] sold by Gattefosse, PhytoCellTecTM Malus Domestica [INCI: Apple (Malus Domestica) Fruit Cell Culture, Xanthan Gum, Glycerin, Lecithin, Phenoxyethanol, Water] sold by Mibelle Biochemistry, Phytosphingosine [INCI: Salicyl Phytosphingosine] sold by Evonik Goldschmidt SLC, Dakaline [INCI: Prunus Amygdalus kernel extract, Anogeissus Leiocarpus bark extract] sold by Soliance, [INCI: Hexapeptide-9], GP4G [INCI: Artemia Extract], D'OrientineTM [INCI: Phoenix Dactylifera (Date) Seed Extract], or EderlineTM [INCI: Apple (Pyrus Malus) (Apple) fruit extract] sold by Vinscience, or Homeostatine [INCI: Enteromorpha Compressa, Caesalpinia Spinosa] sold by Provital.

Alternatively, the aforementioned cosmetic or pharmaceutical compositions of the present invention may contain a cosmetically or pharmaceutically effective amount of at least one synthetic compound, plant extract, or product obtained by biotechnological methods, which is an elastase-inhibiting agents such as, without limitation, [INCI: Glycine Soja (Soybean) Protein], [INCI: Glycine Soja (Soybean) Protein, Oxidoreductase] or -Age [INCI: Hydrolyzed Rice Bran Hydrolyzed], wild soybean (soybean) protein, oxidoreductase] sold by Pentapharm/DSM Glycerin, Juvenesce [INCI: Ethoxydiglycol and Caprylic Triglycerides, Retinol, Ursolic Acid, Vitamin K1, Ilox Ilomastat], MicromerolTM [INCI: Apple (Pyrus Malus) Extract], Heather Extract [INCI: Calluna Vulgaris Extract], [INCI: Hydrolyzed Potato Protein] or FlavagrumTMPEG [INCI : PEG-6 Isostearate, Hesperetin Laurate] sold by Coletica/Engelhard/BASF, TP LS8657 [INCI: Pea (Pisum Sativum) Extract] sold by Laboratoires Serobiologiques/Cognis, Sepilift DPHP [INCI: Dipalm Acyl Hydroxyproline] sold by SEPPIC, [INCI: Alcohol, Water, Glycerin, Hydrolyzed Rice Protein, Ilex Aquifolium Extract, Sodium Ursolicate, Sodium Oleanate]sold by Rahn, Defense 2 [INCI: Juglans Regia (Walnut) Seed Extract] sold by Gattefosse Age, IP 2000 [INCI: Dextran, Trifluoroacetyl Tripeptide-2] sold by IEB and Atrium, Radicaptol [INCI: Propylene Glycol, Water, Passiflora Incarnata Flower (Passiflora Incarnata Flower) Extract, Ribes Nigrum (Black Currant) Leaf Extract, Vitis Vinifera (Grape) Leaf Extract] sold by Solabia or ViaPure^{™} Boswellia [INCI: Olivanum (Boswellia serrata) extract] etc., sold by Soliance.

Alternatively, the above-mentioned cosmetic or pharmaceutical compositions of the present invention may contain a cosmetically or pharmaceutically effective amount of at least one synthetic compound, plant extract, or product obtained by biotechnological methods, which is an active carbonyl species substance scavengers, free radical scavengers and/or anti-glycation agents such as, without limitation, carnosine and its derivatives, GHK [INCI: tripeptide-1] and its salts and/or derivatives, or [INCI: hydrolyzed wheat protein, hydrolyzed soybean protein, tripeptide-1] or PreventheliaTM [INCI: diaminopropionyl tripeptide-33] etc., sold by Lipotec. Optionally, the above-mentioned cosmetic composition or pharmaceutical composition of the present invention may contain a cosmetically or pharmaceutically effective amount of at least one extract, which is an anti-wrinkle agent and/or an anti-aging agent, such as, without limited to, grape, extracts of Rosa canina, Curcuma longa, Iris pallida, Theobroma cacao, Ginkgo biloba, Leontopodium Alpinum or Dunaliellasalina, etc., or at least one synthetic compound or product which is an anti-wrinkle or anti-aging agent such as, without limitation, [INCI: Palmitoyl Pentapeptide-3], Matrixyl [INCI: Palmitoyl Tetrapeptide-7, Palmitoyl Oligopeptide], EssenskinTM [INCI: Calcium Hydroxymethionine], Renovage [INCI: Teprenone] or [INCI: Palmitoyl Oligopeptide] sold by Sederma/Croda, [INCI: Pentapeptide-3], Syn [INCI: Dipeptide Diaminobutyryl Benzylamide Diacetate], -Coll [INCI: Palmitoyl Tripeptide-5], Phytaluronate [INCI: Locust Bean (Carob) Gum] or [INCI: wild soybean (soybean) protein, oxidoreductase] sold by Pentapharm/D SM, MyoxinolTM [INCI: hydrolyzed coffee ambrette (Hibiscus Esculentus) extract], SyniorageTM [INCI: acetyl tetrapeptide-11], DermicanTM [INCI: Acetyl Tetrapeptide-9] or DN-AGETMLS [INCI: Cassia Alata Leaf Extract] sold by Laboratoires Serobiologiques/Cognis, Algisum [INCI: Methylsilanol Mannuronate] or Hydroxyprolisilane [INCI: Methylsilanol Hydroxyproline Aspartate] sold by Exsymol, [INCI: Acetyl Hexapeptide-8], SNAP-7 [INCI: Acetyl Hexapeptide-8], Peptide-4], SNAP-8 [INCI: Acetyl Octapeptide-3], [INCI: Pentapeptide-18], InylineTM [INCI: (recommended) Acetyl Hexapeptide-30], [INCI: Hydrolyzed Wheat Protein, Hydrolyzed Soy Protein, Tripeptide-1], PreventheliaTM [INCI: Diaminopropionyl Tripeptide-33], DecorinylTM [INCI: Tripeptide-10 Citrulline], TrylagenTM [INCI: Pseudo-Alternating Units Pseudoalteromonas Ferment Extract, Hydrolyzed Wheat Protein, Hydrolyzed Soybean Protein, Tripeptide-10 Citrulline, Tripeptide-1]; [INCI: Acetyl Tetrapeptide-5], Peptide AC29 [INCI: Acetyl Tripeptide -30 Citrulline], Lipochroman-6 [INCI: Dimethylmethoxychromanol], ChromabrightTM [INCI: Dimethylmethoxychromanyl Palmitate], VilasteneTM [INCI: Lysine Hydrochloride, Lecithin, Tripeptide-10 Citrulline], dGlyAGETM [INCI: Lysine Hydrochloride, Lecithin, Tripeptide-9 Citrulline] or [INCI: Pseudoalteromonas Ferment Extract] sold by Lipotec, [INCI: Tripeptide-1, Dextran] sole by InstitutEuropeen de BiologieCellulaire, [INCI: Hexapeptide-9], OrsirtineTM GL [INCI: Oryza Sativa (rice) extract], D'OrientineTM IS marketed by Vinscience [INCI: Date Palm (Jujube) Seed Extract], PhytoquintescineTM [INCI: Einkorn (Triticum monococcum) Extract] or QuintescineTM IS [INCI: Dipeptide-4] sold by Vincience, BONT-L Peptide [INCI: Palmitoyl Hexapeptide-19] sole by InfinitecActivos, DeepalineTM PVB [INCI: Palmitoyl Hydrolyzed Wheat Protein] or DPHP [INCI: Dipalmitoyl Hydroxyproline] sold by Seppic, [INCI: Acmella oleracea extract], In-Tense [INCI: Golden button flower extract], or Age Defense 2 [INCI: Juglans Regia (Walnut) seed extract] sold by Gattefossé Expression, ThalassineTM [INCI: Algal Extract] sold by Biotechmarine, ChroNOlineTM [INCI: Hexanoyl Tetrapeptide-3] or Thymulen-4 [INCI: Acetyl Tetrapeptide-2] sold by Atrium Innovations/Unipex Group, EquiStat [INCI: Apple Fruit Extract, Wild Soybean Seed Extract] or Juvenesce [INCI: Ethoxylated Diglycol and Caprylic Triglycerides, Retinol, Ursolic Acid, Vitamin K1, Ilomastat] sold by Coletica/Engelhard/BASF, Ameliox [INCI: Carnosine, Tocopherol, Silybum Marianum Fruit Extract] or PhytoCellTec Malus Domestica [INCI: Apple Fruit Cell Culture] sold by Mibelle Biochemistry, Bioxilift [INCI: Anise (Pimpinella) Anisum) extract] or SMS Anti-[INCI: Anisa apple seed extract], Ca²⁺ channel antagonists such as, without limited to, alverine, manganese or magnesium salts, certain secondary or tertiary amines, retinol and its derivatives, and idebenone (idebenone) and its derivatives, coenzyme Q10 and its derivatives, boswellic acid and its derivatives, GHK and its derivatives and/or salts, carnosine and its derivatives DNA repair enzymes such as, but not limited to, photolyase or T4 endonuclease V, or chloride channel antagonists, etc,. sold by Silab. Alternatively, the aforementioned cosmetic or pharmaceutical compositions of the present invention may contain a cosmetically or pharmaceutically effective amount of at least one synthetic compounds, plant extracts, or products obtained by biotechnological methods, which are agents that stimulates healing, agents that assist healing, agents that stimulate re-epithelialization, and/or agents that assist re-epithelialization, such as, without limited to, Centella asiatica extract, Rosa moschata extract, Echinacea angustifolia Echinacea angustifolia extract, Symphytum ofticinal extract, Equisetum arvense extract, Hypericum perforatum extract, Mimosa tenuitlora extract, Aloe vera vera) extract, Epithelizing [INCI: Calendula (Calendula officinalis), Hypericum, Matricaria (Chamomilla recutita), Rosemary (Rosmarinus ofticinalis)] sold by Provital, LS9028 [INCI: hydrolyzed casein, hydrolyzed yeast protein, lysine hydrochloride] sold by Laboratories Serobiologiques/Cognis, [INCI: Zea mays (corn) grain extract], cadherins, integrins, selection proteins, hyaluronan receptors, immunoglobulin, fibroblast growth factor, connective tissue growth factor, platelet-derived growth factor, vascular endothelial growth factor, epidermal growth factor, insulin-like growth factor, keratinocyte growth factor, colony stimulating factor, transforming growth factor-beta, tumor necrosis factoralpha, interferon, interleukin, matrix metalloproteinase, protein tyrosine phosphatase receptor sold by Coletica/Engelhard/BASF, [INCI: Pseudoalteromonas ferment extract] or [INCI: tripeptide-10 citrulline], or a mixture thereof sold by Lipotec.

Furthermore, the afore-mentioned cosmetic or pharmaceutical compositions of the present invention may contain or may be co-administered with analgesic and/or anti-inflammatory compounds to reduce swelling and irritation associated with sensitive skin and healing process or treat overgrown scars or scabs. Among said compounds, emphasis may be placed on steroid-type compounds such as hydrocortisone, non-steroid-type compounds such as acetaminophen or acetylsalicylic acid, or natural extracts or essential oils with inherent analgesic and anti-inflammatory activities.

Optionally, the formulation of said cosmetic or pharmaceutical compositions is selected from creams, oils, milks, balms, foams, lotions, gels, liniments, sera, soaps, shampoos, conditioners, serums, ointments, mousses, pomades, powders, bars, pencils, sprays, aerosols, capsules, tablets, granules, chewing gums, solutions, suspensions, emulsions, syrups, elixirs, polysaccharide films, jellies or gelatins;

Optionally, the capsules include: soft capsules, hard capsules, optionally gelatin capsules; Optionally, the tablets include: sugar-coated tablets.

The cosmetic or pharmaceutical compositions of the present invention may include transdermal enhancers that increase the absorption of the peptides of the present invention, such as, without limited to dimethylsulfoxide, dimethylacetamide, dimethylformamide, surfactants, azone (1-dodecylazacycloheptane-2-one), alcohol, urea, ethoxydiglycol, acetone, propylene glycol or polyethylene glycol, etc.

In addition, the peptide represented by formula (I) of the present invention, or its stereoisomer, or a mixture of stereoisomers, or its cosmetically acceptable salt, or its pharmaceutically acceptable salt, or the cosmetic or pharmaceutical compositions that can be prepared by iontophoresis, sonophoresis, electroporation, microelectropatch, mechanical compression, osmotic pressure gradient, dressing therapy, microinjection or needle-free injection with the aid of pressure (e.g. by injections under oxygen pressure), or any combination thereof, are administered to the localized area to be treated in order to achieve greater penetration of the peptides of the invention. The area of administration will be determined by the nature of the condition, disorder and/or disease to be treated and/or cared for.

In addition, the peptide represented by formula (I) according to the present invention, or its stereoisomer, or a mixture of stereoisomers, or its cosmetically acceptable salt, or its pharmaceutically acceptable salt, or the cosmetic or pharmaceutical compositions described above may be incorporated into any form of functional food or fortified food, such as, without limited to, dietary bars or compressed or non-compressed powders. These powders can be dissolved in water, juice, soda, dairy products, soy derivatives, or can be incorporated into dietary bars. The peptides of the invention can be formulated with common excipients or adjuvants used in oral compositions or food supplements, such as, without limited to, fatty components, aqueous components, humectants, preservatives, thickeners, flavorings, fragrances, antioxidants, or coloring agents.

The peptides of the invention have variable solubility in water depending on the nature of their sequence or any possible modification in the N-terminus and/or C-terminus. The peptides of the present invention can thus be incorporated into compositions by aqueous solutions, and those that are insoluble in water can be dissolved in cosmetically or pharmaceutically acceptable conventional solvents such as, without limited to ethanol, propanol, isopropanol, propylene glycol, glycerin, butylene glycol, or polyethylene glycol, or any combination thereof.

The cosmetically or pharmaceutically effective amount of the peptides of the invention to be administered, as well as their dosage, will depend on many factors, including age, the state of the patient, the severity of the condition or disease, the route and frequency of administration, and the specific nature of the peptide to be used.

"Cosmetically or pharmaceutically effective amount" means a non-toxic amount of one or more peptides of the invention which is sufficient to provide the desired effect. The peptides of the invention are used in the cosmetic or pharmaceutical compositions of the invention at a cosmetically or pharmaceutically effective concentration to obtain the desired effect; in a preferred form, at 0.00000001% by weight and 20% by weight, preferably between 0.000001% by weight and 15% by weight, more preferably between 0.0001% by weight and 10% by weight, and even more preferably between 0.0001% by weight and 5% by weight.

In another aspect of the present invention, there is provided a cosmetically or pharmaceutically acceptable delivery system or sustained release system in order to achieve better penetration of the active ingredient and/or to improve its pharmacokinetic and pharmacodynamic properties, which Comprising an effective amount of the peptide represented by the above-mentioned formula (I), or its stereoisomer, or a mixture of stereoisomers, or its cosmetically acceptable salt, or its pharmaceutically acceptable salt, or the above-mentioned cosmetic or pharmaceutical compositions;

The term "delivery system" refers to a diluent, adjuvant, excipient or carrier with which the peptides of the invention are administered, selected from: water, oil or surfactants, including petroleum sources, animal sources, plant sources, or Those of synthetic origin, such as, without limited to peanut oil, soybean oil, mineral oil, sesame oil, castor oil, polysorbates, sorbitan esters, ether sulfates, sulfates, betaines, glucosides, maltosides, fatty Alcohol, Nonoxynol, Poloxamer, Polyoxyethylene, Macrogol, Dextrose, Glycerin, Digitonin and the like. Those of ordinary skill in the art know the diluents that can be used in the different delivery systems in which the peptides of the invention can be administered.

The term "sustained release" is used in the conventional sense to refer to a delivery system that provides a gradual release of the compound over a period of time, and preferably, but not necessarily, a relatively constant level of compound release over the entire period of time. Examples of delivery systems or sustained release systems are liposomes, oleosomes, non-ionic surfactant liposome vesicles, ethosomes, millicapsules, microcapsules, nanocapsules, nanostructured lipid carriers, sponges, cyclodextrins, lipid vesicles, micelles, millispheres, microspheres, nanospheres, lipospheres, microemulsions, nanoemulsions, milliparticles, microparticles or nanoparticles. Preferred delivery systems or sustained release systems are liposomes and microemulsions, more preferably water-in-oil microemulsions with an internal structure of reverse micelles.

Sustained release systems can be prepared by methods known in the art and can be administered, for example, by topical or transdermal administration, including adhesive patches, non-adherent patches, occlusive patches, and microelectronic patch; or by systemic administration such as and without limitation, oral or parenteral routes, including nasal, rectal, subcutaneous implantation or injection, or direct implantation or injection into specific body parts, and preferably should A relatively constant amount of these peptides of the invention is released. The amount of peptide contained in the sustained release system will depend, for example, on the site where the composition is to be administered, the release kinetics and duration of the peptide of the invention, and the condition, disorder and/or condition to be treated and/or cared for. the nature of the disease.

Another aspect of the present invention provides a cosmetically or pharmaceutically acceptable solid organic polymer or solid inorganic support, which contains an effective amount of the peptide represented by the above formula (I), or its stereoisomer, or a mixture of stereoisomers, or a cosmetically acceptable salt thereof, or a pharmaceutically acceptable salt thereof, or a cosmetic or pharmaceutical compositions as described above; selected from, but not limited to: talc, bentonite, silica, starch, or maltodextrin, etc.

Another aspect of the present invention provides a cosmetic product comprising an effective amount of the above-mentioned peptide represented by formula (I), or its stereoisomer, or a mixture of stereoisomers, or a cosmetically acceptable salt thereof, or a pharmaceutically acceptable salt thereof, or the aforementioned cosmetic or pharmaceutical compositions; Optionally, the cosmetic is selected from: concealers, foundations, makeup removers, makeup remover milks, eye shadows, lip sticks, lip glosses, lip balms or lip powders.

Another aspect of the present invention provides a fabric, non-woven fabric or medical device, which comprises an effective amount of the above-mentioned peptide represented by formula (I), or a stereoisomer or a mixture of stereoisomers thereof, or A cosmetically acceptable salt thereof, or a pharmaceutically acceptable salt thereof, or the aforementioned cosmetic or pharmaceutical compositions. The peptides of the invention are released due to biodegradation of the fabric or non-woven fabric or the anchoring system of the medical device or by friction with the body, by body moisture, by the pH of the skin or by body temperature. In addition, fabrics and nonwovens can be used to make clothes that come into direct contact with the body.

Examples of fabrics, nonwovens, clothing, medical devices and means of immobilizing peptides to them include the above-mentioned delivery systems and/or sustained release systems, which can be found in the literature and are known in the prior art [Schaab C.K. (1986)"Impregnating Fabrics With Microcapsules (using microcapsules to impregnate fabrics)", HAPPI May 1986; Nelson G. (2002) "Application of microencapsulation in textiles (microencapsulation in textiles)" Int. J. Pharm. 242: 5562; "Biofunctional Textiles and the Skin" (2006) Curr. Probl. Dermatol. v.33, edited by Hipler U.C. and Elsner P. S. Karger A.G., Basel, Switzerland; Malcom R.K.; McCullagh S.D., Woolfson A.D., Gorman S.P., Jones D.S., and Cuddy J. (2004) "Controlled release of a model antibacterial drug from a novel self lubricating silicone (from a novel self lubricating silicone Controlled release of antimicrobial drug models from lubricated silicone biomaterials)" J.Cont.Release97:313320]. Preferred fabrics, nonwovens, clothing or medical devices are bandages, gauzes, t-shirts, socks, pantyhoses, underwears, girdles, gloves, diapers, sanitary napkins, dressings, bedspreads, wipes, adhesive patches, non-adhesive patches, occlusive patches, microelectronic patches or masks.

Another aspect of the present invention provides a use of peptide represented by the above formula (I), or its stereoisomer, or a mixture of stereoisomers, or its cosmetically acceptable salt, or its pharmaceutically acceptable salts, or cosmetic or pharmaceutical compositions described above, or cosmetically or pharmaceutically acceptable delivery systems or sustained release systems described above, or cosmetically or pharmaceutically acceptable solid organic polymers or solid inorganic supports described above, or the above-mentioned cosmetics, or the above-mentioned fabric, non-woven fabric or medical device in the preparation of a cosmetic or pharmaceutical composition for the treatment or care of the skin or mucosa.

Another aspect of the present invention provides a use of peptide represented by the above formula (I), or its stereoisomer, or a mixture of stereoisomers, or its cosmetically acceptable salt, or its pharmaceutically acceptable salts, or cosmetic or pharmaceutical compositions described above, or cosmetically or pharmaceutically acceptable delivery systems or sustained release systems described above, or cosmetically or pharmaceutically acceptable solid organic polymers or solid inorganic supports described above, or the above-mentioned cosmetics, or the above-mentioned fabric, non-woven or medical devices in the preparation of cosmetic compositions or pharmaceutical compositions for treating or nursing body surface wounds, burns, skin ulcers, reducing scar generation, or accelerating scar repair.

Another aspect of the present invention provides a use of peptide represented by the above formula (I), or its stereoisomer, or a mixture of stereoisomers, or its cosmetically acceptable salt, or its pharmaceutically acceptable salts, or cosmetic or pharmaceutical compositions described above, or cosmetically or pharmaceutically acceptable delivery systems or sustained release systems described above, or cosmetically or pharmaceutically acceptable solid organic polymers or solid inorganic supports described above, or the above-mentioned cosmetics, or the above-mentioned fabric, nonwoven or medical devices in the preparation of cosmetic or pharmaceutical compositions for promoting fibroblast proliferation, increasing collagen production, for improving skin barrier function, or for re-epithelializaing or healing skin or mucosa.

One aspect of the present invention provides a use of peptide represented by the above formula (I), or its stereoisomer, or a mixture of stereoisomers, or its cosmetically acceptable salt, or its pharmaceutically acceptable or the above-mentioned cosmetic or pharmaceutical composition, or the above-mentioned cosmetically or pharmaceutically acceptable delivery system or sustained-release system, or the above-mentioned cosmetically or pharmaceutically acceptable solid organic polymer or solid inorganic support, or the above-mentioned cosmetics, or the use of the above-mentioned fabric, nonwoven or medical devices in the preparation of cosmetic compositions or pharmaceutical compositions for treating, preventing or repairing skin aging or photoaging.

Optionally, the treatment, prevention, or repair of skin aging or photoaging is the reduction, prevention or treatment of facial wrinkles.

Another aspect of the present invention provides a method for treating or caring for skin or mucosa, the method comprising administering a cosmetically or pharmaceutically effective amount of the above-mentioned peptide represented by formula (I), or a stereoisomer thereof, or a mixture of stereoisomers, or a cosmetically acceptable salt thereof, or a pharmaceutically acceptable salt thereof, or the afore-mentioned cosmetic or pharmaceutical compositions. Another aspect of the present invention provides a method for treating or nursing body surface wounds, burns, skin ulcers, reducing scar production, and accelerating scar repair, the method comprising administering a cosmetically or pharmaceutically effective amount of the above-mentioned formula (I), or a stereoisomer thereof, or a mixture of stereoisomers, or a cosmetically acceptable salt thereof, or a pharmaceutically acceptable salt thereof, or the aforementioned cosmetic or pharmaceutical compositions.

In another aspect of the present invention, there is provided a method for promoting fibroblast proliferation, increasing collagen production, for improving skin barrier function, or for re-epithelializing or healing skin and/or mucosa, the method comprising administering a cosmetically or pharmaceutically effective amount of the peptide represented by the above formula (I), or its stereoisomer, or a mixture of stereoisomers, or its cosmetically acceptable salt, or its pharmaceutically acceptable salt, or the aforementioned cosmetic or pharmaceutical compositions.

Another aspect of the present invention provides a method for treating, preventing, or repairing skin aging or photoaging, the method comprising administering a cosmetically or pharmaceutically effective amount of the above-mentioned peptide represented by formula (I), or its stereo isomers, or mixtures of stereoisomers, or cosmetically acceptable salts thereof, or pharmaceutically acceptable salts thereof, or the aforementioned cosmetic or pharmaceutical compositions.

The treatment, prevention, or repair of skin aging or photoaging is the reduction, prevention, or treatment of facial wrinkles.

Compositions containing the peptide represented by the above-mentioned formula (I) of the present invention, or its stereoisomer, or a mixture of stereoisomers, or its cosmetically acceptable salt, or its pharmaceutically acceptable salt, or the above-mentioned cosmetic or pharmaceutical compositions may be applied to the skin and/or mucosa, or administered orally or parenterally as required for the treatment and/or care of a condition, disorder and/or disease. The frequency of administration or dosing can vary widely depending on the needs of each subject, with suggested administration or dosing ranging from 1 time per month to 10 times per day, preferably from 1 time per week to 4 times per day, More preferably from 3 times a week to 3 times a day, even more preferably 1 or 2 times a day.

### Definition

To facilitate understanding of the present invention, some terms used in the present invention and the meaning of expressions are explained as follows:
In the present invention, the term "skin" is understood to mean the layers that collectively constitute the skin, from the uppermost layer or stratum corneum to the lowermost layer or subcutaneous tissue, both ends inclusive. These layers are composed of different types of cells, such as keratinocytes, fibroblasts, melanocytes, and/or adipocytes, among others. In the present invention, the term "skin" includes the scalp.

The term "treatment" refers to the administration of the peptides according to the present invention to alleviate or eliminate a disease or condition, or to reduce or eliminate one or more symptoms associated with such a disease or condition. The term "treatment" also encompasses the ability to alleviate or eliminate the physiological consequences of the disease or disorder.

The term "care" includes the prevention of diseases and/or conditions.

The term "prevention" refers to the ability of the peptides of the invention to prevent, delay, or impede the onset or development of a disease or condition before it occurs.

The term "aging" refers to the changes the skin undergoes as it ages (natural aging), or through exposure to sunlight (photoaging) or exposure to environmental pollutants such as chemical dirt or pollutants, tobacco smoke, etc. changes, and includes all externally visible and/or perceptible changes by touch, such as, without limited to: the development of discontinuities in the skin (such as wrinkles, fine lines, expression lines, stretch marks, striae, furrows wrinkles, unevenness or roughness, increased pore size, loss of moisture, loss of elasticity, loss of firmness, loss of smoothness, loss of ability to recover from deformation, loss of resilience), sagging of the skin (such as sagging cheeks, bags under the eyes, or double chin, etc.), changes in skin color (such as scarring, redness, bags under the eyes, or areas of hyperpigmentation such as age spots or freckles), abnormal differentiation, hyperkeratinization, elastosis, keratosis, hair loss, orange Dermoid skin, loss of collagen structure, and other histologic changes in the stratum corneum, dermis, epidermis, vasculature (eg, development of spider veins or telangiectasia), or those tissues adjacent to the skin.

The term "photoaging" refers to premature aging of the skin due to long-term exposure of the skin to ultraviolet radiation, which exhibits the same physiological characteristics as natural aging, such as, without limited to: sagging, sagging, color changes or hyperpigmentation Regular, abnormal and/or hyperkeratosis.

In this specification, the abbreviations used for amino acids follow the rules specified by the IUPAC-IUB Commission of Biochemical Nomenclature in the European Journal of Biochemistry (Eur.J.Biochem.1984, 138:9-37).

Thus, for example, Ala represents NH₂-CH(CH₃)-COOH, Ala- represents NH₂-CH(CH₃)-CO-, - Ala represents -NH-CH(CH₃)-COOH, and -Ala- Represents -NH-CH(CH₃)-CO-. Thus, the hyphen denoting a peptide bond eliminates the OH in the 1-carboxyl group of an amino acid (represented here in conventional non-ionized form) when located to the right of the hyphen, and eliminates the H in the amino acid 2-amino when located to the left of the hyphen; both modifications can be applied to the same symbol (see Table 1).

**Table 1 Structure of amino acid residues and their one-letter and three-letter abbreviations**

| name/abbreviations | amino acid residues | name/abbreviations | amino acid residues |
|---|---|---|---|
| Phenylalanyl -Phe- F | 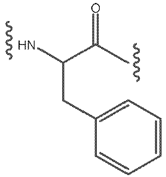 | Prolyl -Pro-P | 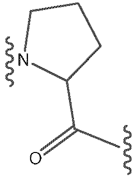 |
| Arginyl -Arg- R | 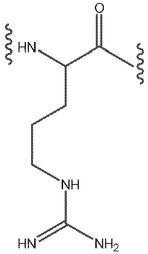 | Methionyl -Met-M | 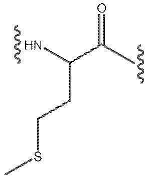 |
| Glycyl -Gly- G | 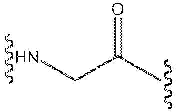 | Isoleucyl -Ile-I | 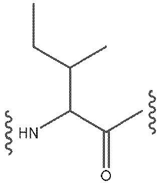 |
| Cysteinyl -Cys- C | 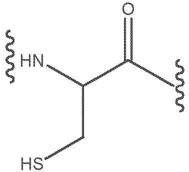 | Threonyl -Thr-T | 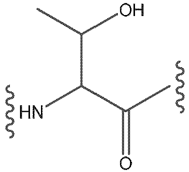 |
| Alanyl -Ala- A | 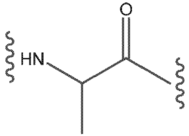 | Asparaginyl -Asn-N | 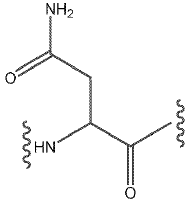 |

The abbreviation "Ac-" is used in the present invention to denote the acetyl group (CH₃-CO-), and the abbreviation "Palm-" is used to denote the palmitoyl group (CH₃ -(CH₂)₁₄-CO-).

The beneficial effect that the present invention obtains with compared to prior art comprises:
The peptide described in the present invention is obtained by artificial design, is easy to synthesize, can promote the proliferation of fibroblasts, improve the skin barrier function, is beneficial to promote wound healing, treat or care for body surface wounds, burns, skin ulcers, reduce scar generation, and accelerate scar repair, and also useful for treating, preventing and/or repairing skin aging and/or photoaging.

### Description of Drawings

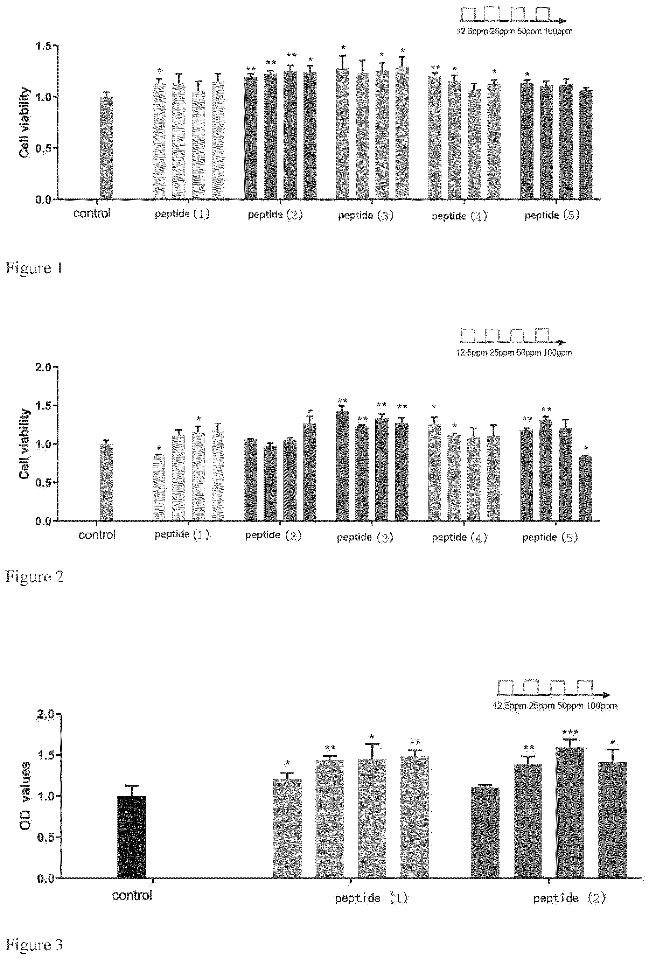

Fig. 1 is a graph showing the effects of peptides (1) to (5) on the activity of HaCaT cells. * indicates that there is a statistical difference between the administration group and the blank control group, p<0.05 (n=4). ** indicates that there is a significant difference between the administration group and the blank control group, p<0.01 (n=4).
Fig. 2 is a graph showing the effect of peptide (1) to peptide (5) on the activity of NIH3T3 cells. * indicates that there is a statistical difference between the administration group and the blank control group, p<0.05 (n=4). ** indicates that there is a significant difference between the administration group and the blank control group, p<0.01 (n=4).
Fig. 3 is a graph showing the effect of peptide (1) and peptide (2) on the adhesion of HaCaT cells. * indicates that there is a statistical difference between the administration group and the blank control group, p<0.05 (n=4). ** indicates that there is a significant difference between the administration group and the blank control group, p<0.01 (n=4). *** indicates that there is a extremely significant difference between the administration group and the blank control group, p<0.001 (n=4).

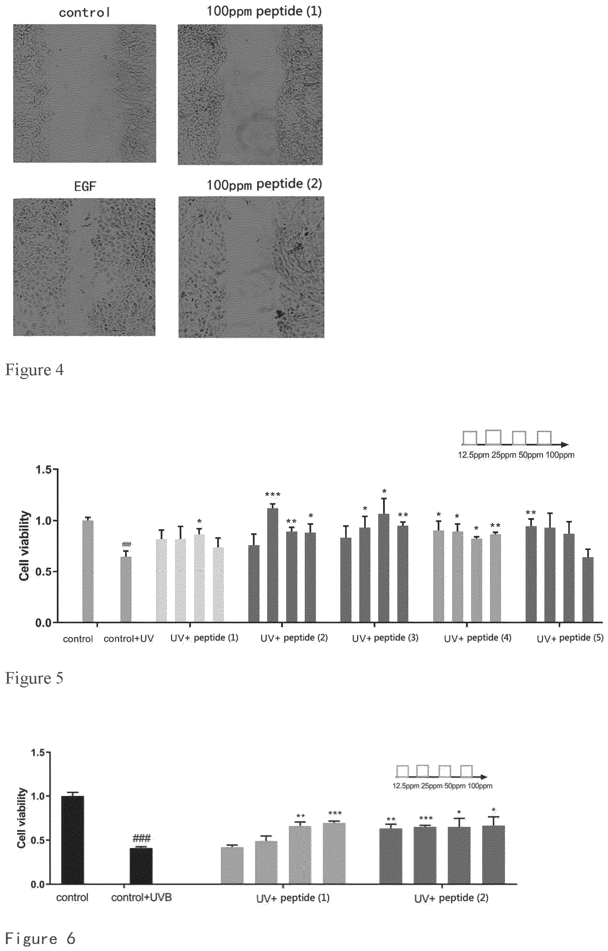

Fig. 4 is a graph showing the results of the scratch experiment on HaCaT cells.
Fig. 5 is a graph showing the repairing effect of peptide (1) to peptide (5) on light-damaged HaCaT cells. ### indicates that there is a extremely significant difference as compared with non-irradiated normal control group, p<0.001 (n=4). * indicates that there is a statistical difference as compared with the control group after radiation, p<0.05 (n=4). ** indicates that there is a significant difference compared with the control group after radiation, p<0.01 (n=4). *** indicates that there is a extremely significant difference as compared with the control group after radiation, p<0.001 (n=4).
Fig. 6 is a graph showing the repairing effect of peptide (1) and peptide (2) on photodamaged NIH3T3 cells. ### indicates that there is a extremely significant difference as compared with non-irradiated normal control group, p<0.001 (n=4). * indicates that there is a statistical difference as compared with the control group after radiation, p<0.05 (n=4). ** indicates that there is a significant difference as compared with the control group after radiation, p<0.01 (n=4). *** indicates that there is a extremely significant difference as compared with the control group after radiation, p<0.001 (n=4).

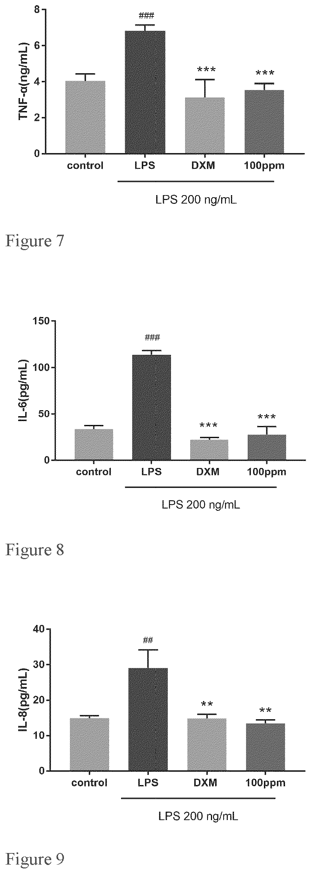

Fig. 7 is a graph showing the effect of peptide (2) on TNF-α secretion. ### indicates that there is a extremely significant difference as compared with the control group, p<0.001 (n=4). *** indicates that there is a extremely significant difference as compared with LPS group, p<0.001 (n=4).
Fig. 8 is a graph showing the effect of peptide (2) on IL-6 secretion. ### indicates that there is a extremely significant difference as compared with the control group, p<0.001 (n=4). *** indicates that there is a extremely significant difference as compared with LPS group, p<0.001 (n=4).
Fig. 9 is a graph showing the effect of peptide (2) on IL-8 secretion. ## indicates that there is a significant difference as compared with the control group, p<0.01 (n=4). ** indicates that there is a significant difference as compared with the LPS group, p<0.01 (n=4).

### Embodiments

In order to better understand the present invention, the present invention will be described in detail below in conjunction with embodiment and accompanying drawing, however, it should be understood that these embodiments and accompanying drawing are only used for illustration purposes and are not intended to limit the scope of the present invention.

### Abbreviation

The abbreviations used for amino acids follow the rules specified by the Biochemical Nomenclature Commission of IUPAC-IUB in Eur J. Biochem. (1984) 138:9-37 and J. Chem (1989) 264:633-673.

Wang Resin: Wang resin; DMF: N,N-dimethylformamide, DCM: dichloromethane; DIC: diisopropylcarbodiimide; Ac₂O: acetic anhydride; DIPEA: diisopropylethylamine; Fmoc: 9-fluorenylmethoxycarbonyl; piperidine: piperidine; HOBt: 1-hydroxybenzotriazole; TFA: trifluoroacetic acid; TIS: triisopropylsilane; EDT: 1,2-ethanedisulfide Alcohol; DMSO: Dimethyl Sulfoxide; Ac: Acetyl; Palm: Palmitoyl; Ala: Alanine; Pro: Proline; Phe: Phenylalanine; Arg: Arginine; Met: Methionine; Gly: Glycine; Ile: Isoleucine; Cys: Cysteine; Thr: Threonine; Asn: Asparagine; Pbf: (2,2,4,6,7-pentamethyl-2,3-dihydro-1-benzofuran-5-yl)sulfonyl; Trt: Triphenylmethyl or Trityl; tBu: tert-butyl; Amide Resin: a starting resin for polypeptide synthesis; Fmoc-linker: 4 -[(2,4-Dimethoxyphenyl)(Fmoc-amino)methyl]phenoxyacetic acid.

### Example 1

Preparation of Fmoc-Ala-Pro-Phe-Arg(Pbf)-Met-Gly-Ile-Cys(Trt)-Thr(tBu)-Thr(tBu)-Asn(Trt)-Wang Resin.

### 1.1 Resin swelling

Weigh 35g (49.7mmol) of Wang Resin into a 2L solid-phase synthesis reaction column. Add 100ml of DMF to completely immerse the resin, then enter nitrogen gas, turn on the nitrogen gas stiring and blowing for 5 minutes, and then drain the solvent for 5 minutes. Continue to add 100ml of DMF, completely immerse the resin, and enter nitrogen gas for 30 minutes, the resin is completely swollen, and the solvent is drained. Then add 100ml of DMF, completely immerse the resin and then enter nitrogen gas, turn on the nitrogen gas stiring and blowing, drain the solvent for 5 minutes, and the swelling is over.

### 1.2 Feeding reaction

Weigh 53.38g of Fmoc-Asn(Trt)-OH (89.46mmol; 1.8 equiv), and add 14.51g of HOBt into a dry 500ml round-bottomed ground flask. Add DMF to dissolve it, and put it in a refrigerator at ±5°C for 10 minutes. Add 20.78ml DIC to activate for 10min, avoid water vapor. The activated amino acid was added to the resin to react for 2 hours, the reaction temperature was controlled at 25° C to 35° C, and the reaction liquid was removed. Wash the resin with DMF, 100ml each time, mix the resin and solvent evenly, and then stir for 2min by timing, and drain the solvent. The resin was washed several times with DMF until the solvent filtered out with suction was clear and transparent.

The degree of substitution of the resin was detected to be 0.5, and the calculated synthesis scale was 24.9 mmol.

The N-terminal Fmoc group was deprotected, 24.74 g of activated Fmoc-Thr(tBu)-OH (62.25 mmol; 2.5 equiv.) was coupled onto the peptidyl resin using DMF as solvent in the presence of 10.09 g HOBt and 14.46 ml DIC, the reaction was continued for 2h. The resins were then washed and the deprotection treatment of the Fmoc group was repeated for coupling of the next amino acid. In each coupling, 24.74 g of Fmoc-Thr(tBu)-OH (62.25 mmol; 2.5 eq.); 36.46 g of Fmoc-Cys(Trt)-OH (62.25 mmol; 2.5 eq); 22 g ofFmoc-Ile-OH (62.25 mmol; 2.5 eq); 18.51 g ofFmoc-Gly-OH (62.25 mmol; 2.5 eq) were coupled sequentially using DMF as solvent in the presence of 10.09 g HOBt and 14.46 ml DIC.

The N-terminal Fmoc group was deprotected, 32.37 g of activated Fmoc-Met-OH (87.15 mmol; 3.5 equiv) was coupled onto the peptidyl resin using DMF as solvent in the presence of 14.13 g HOBt and 20.24 ml DIC, the reaction was continued for 2h. The resins were then washed and the deprotection treatment of the Fmoc group was repeated for coupling of the next amino acid. In each coupling, 56.54 g ofFmoc-Arg(Pbf)-OH (87.15 mmol; 3.5 equiv); 33.76 g of Fmoc-Phe-OH (87.15 mmol; 3.5 equiv); 29.40 g of Fmoc-Pro-OH (87.15 mmol; 3.5 equiv) and then 27.13 g of Fmoc-Ala-OH (87.15 mmol; 3.5 equiv) were coupled sequentially using DMF as solvent in the presence of 14.13 g HOBt and 20.24 ml DIC.

After the synthesis, the resin was washed with DMF, 100 ml each time, the resin and the solvent were uniformly mixed, stirred for 2 minutes, and the solvent was drained. The resin was washed several times with DMF until the solvent filtered out with suction was clear and transparent.

The process of De Fmoc; De-Fmoc twice with 500ml 20% piperidine/DMF, the first reaction was 5 minutes, the second reaction was 8 minutes, and the solvent was drained for 3 minutes. Wash the resin 7-8 times with DMF, and remove the solvent.

### Example 2

Preparation of Fmoc-Ala-Pro-Phe-Arg(Pbf)-Met-Gly-Ile-Cys(Trt)-Thr(tBu)-Thr(tBu)-Asn(Trt)-Linker-Amide Resin.

### 2.1 Preparation of Fmoc-Linker-Amide Resin

Weigh 50 g (60 mmol) of Amide Resin into a 2L solid-phase synthesis reaction column. Wash the resin once with 400ml DMF, swell the resin with 500ml DCM for 20min, and remove the solvent. Wash the resin 3 times with 400ml DMF.

Weigh 80.9g (150mmol) of Fmoc-linker and 24.3g (180mmol) of HOBt into a dry 500ml wide-mouth Erlenmeyer flask. Add about 150ml of DMF solvent to dissolve and then place in an ice-water bath to cool for 2 minutes, add DIC 30ml (195mmol) to activate for 3 minutes, avoiding water vapor. The activated Fmoc-linker was added to the swollen resin to react for 2 hours, and the reaction solution was removed. Wash the resin 3 times with 400ml DMF, 2min each time. After adding the mixture of 28ml (300mmol) of Ac₂O, 10.7ml (60mmol) of DIPEA and 400ml of DMF to seal for 3 hours, the resin was washed four times with 500ml of DMF and twice with 500ml of DCM. Remove the solvent.

### 2.2 De-Fmoc

Fmoc-Linker-Amide Resin was de-Fmoc twice with 500ml 20% piperidine/DMF, the first reaction was 5 minutes, the second reaction was 8 minutes, and the solvent was drained for 3 minutes. Wash resin 4 times with 500ml DMF, 2 times with 500ml DCM. Remove the solvent.

### 2.3 Feeding reaction

Weigh 52.21 g of Fmoc-Asn(Trt)-OH (87.5 mmol), and add 14 g of HOBt into a dry 500 ml wide-mouth Erlenmeyer flask. DMF was added to dissolve it, and cooled in an ice-water bath. Add 20ml DIC to activate for 3min, avoid water vapor. The activated amino acid was added to the deprotected resin to react for 2 hours, and the reaction solution was removed. Wash the resin twice with 500ml DMF, 2min each time, wash the resin twice with 400ml DCM, and remove the solvent.

The N-terminal Fmoc group was deprotected, 34.78 g of activated Fmoc-Thr(tBu)-OH (87.5 mmol) was coupled onto the peptidyl resin in the presence of 14 g HOBt and 20 ml DIC using DMF as solvent, the reaction was continued for 2h. The resins were then washed and the deprotection treatment of the Fmoc group was repeated for coupling of the next amino acid. In each coupling, 34.78 g of Fmoc-Thr(tBu)-OH (87.5 mmol); 51.25 g of Fmoc-Cys (Trt)-OH (87.5 mmol); 30.92 g of Fmoc-Ile-OH (87.5 mmol); 26.01 g of Fmoc-Gly-OH (87.5 mmol); 32.50 g of Fmoc-Met-OH (87.5 mmol); 56.77 g of Fmoc-Arg(Pbf)-OH (87.5 mmol); 33.90 g of Fmoc-Phe-OH (87.5 mmol); 29.52 g of Fmoc-Pro-OH (87.5 mmol) and then 27.24 g of Fmoc-Ala-OH (87.5 mmol).

After this synthesis, the resin was washed 4 times with 600 ml DMF for 2 min each, 2 times with 500 ml DCM and the solvent was removed.

### Example 3

The N-terminal Fmoc protecting group was removed.

Deprotect the N-terminal Fmoc groups of these peptidyl resins obtained in embodiment 1 and embodiment 2, use 500ml 20% piperidine/DMF to remove Fmoc twice, first reaction 5min, second reaction 8min, drain the solvent for 3 min. Wash the resin 4 times with 600ml DMF, 2min each time, wash the resin 2 times with 500ml DCM, and remove the solvent.

### Example 4

The R₁ palmitoyl group was introduced onto the peptidyl resin obtained in Example 3.

In the presence of 1.53 g of HOBt (10 mmol; 10 equiv) and 1.54 mL of DIC(10 mmol; 10 equiv), 2.56 g of palmitic acid (10 mmol; 10 equiv) pre-dissolved in 1 ml of DMF was added to 1 mmol of the peptidyl resin obtained in Example 3. They were allowed to react for 15 h, after which the resin was washed 4 times with 600 ml DMF for 2 min each, 2 times with 500 ml DCM, and the solvent was removed.

### Example 5

The R₁ acetyl group was introduced onto the peptidyl resin obtained in Example 3. 1 mmol of the peptidyl resin obtained in Example 3 was treated with 25 equiv of Ac₂O using 5 mL of DMF as solvent in the presence of 25 equiv of DIPEA. They were allowed to react for 30 min, after which the resin was washed 4 times with 600 ml DMF for 2 min each, 2 times with 500 ml DCM, and the solvent was removed.

### Example 6

The peptidyl resins obtained in Examples 3, 4, and 5 were cleaved from the polymer support. The peptidyl resins obtained in Examples 3, 4, and 5 were shrunk three times with 500 ml, 300 ml, and 200 ml of methanol. Vacuum dry.

Weigh 1 g of the obtained dried peptidyl resins of Examples 3, 4, and 5, and treat with 7 ml of lysate (TFA: EDT: anisole: thioanisole: TIS: H₂O=87.5:2.5:2.5:2.5:2.5:2.5) for 2.5 hours. Suction filter with a sand core funnel, wash the resin with 1ml of trifluoroacetic acid, slowly add the filtrate dropwise to 64ml of frozen isopropyl ether, a large amount of white solid precipitates, wash with isopropyl ether for 6 times after centrifugation. The final precipitate was dried under vacuum.

### Example 7

Purification was carried out by high performance liquid chromatography.

The crude peptide obtained in Example 6 was dissolved with methanol and water, and filtered with a filter membrane with a pore size of 0.45 µm.

Purification preparation conditions:
Mobile phase A: pure acetonitrile; mobile phase B: 0.1% acetic acid aqueous solution;
Detection wavelength: 215nm;
Chromatographic column: C18-20*150mm;

Elution gradient:

| Time (min) | Flow rate (ml/min) | A% | B% |
|---|---|---|---|
| 0 | 40 | 10 | 90 |
| 3 | 40 | 12 | 88 |
| 63 | 40 | 37 | 63 |
| 64 | 40 | 10 | 90 |
| 75 | 40 | 10 | 90 |

The filtered sample was injected and purified, the fractions were collected, concentrated and freeze-dried to obtain the following peptides with a purity of over 90%:
1. H-Ala-Pro-Phe-Arg-Met-Gly-Ile-Cys-Thr-Thr-Asn-OH;
2. H-Ala-Pro-Phe-Arg-Met-Gly-Ile-Cys-Thr-Thr-Asn-NH₂;
3. Palm-Ala-Pro-Phe-Arg-Met-Gly-Ile-Cys-Thr-Thr-Asn-OH, namely peptide (8);
4. Palm-Ala-Pro-Phe-Arg-Met-Gly-He-Cys-Thr-Thr-Asn-NH₂, namely peptide (9);
5. Ac-Ala-Pro-Phe-Arg-Met-Gly-Ile-Cys- Thr- Thr-Asn-OH, namely peptide (10);
6. Ac-Ala-Pro-Phe-Arg-Met-Gly-Ile-Cys-Thr-Thr-Asn-NH₂, namely peptide (11).

### Example 8

The peptide obtained in Example 7 was oxidized.

The peptide obtained in Example 7 was added to a 250ml pear-shaped bottle, dissolved in 80ml of purified water, 8g of guanidine hydrochloride and 20ml of DMSO were added, stirred for 30h openly, and the reaction process was monitored by HPLC. After oxidation, add 4 ml of acetic acid to dissolve the precipitate, and vacuum filter it with a 0.22 µm filter membrane.

### Example 9

The filtrate obtained in Example 8 was purified by high performance liquid chromatography.

Purification preparation conditions:
Mobile phase A: pure acetonitrile; mobile phase B: 0.1% acetic acid aqueous solution;
Detection wavelength: 215nm;
Chromatographic column: C18-20*150mm;

Elution gradient:

| Time (min) | Flow rate (ml/min) | A% | B% |
|---|---|---|---|
| 0 | 40 | 8 | 92 |
| 5 | 40 | 8 | 92 |
| 50 | 40 | 53 | 47 |
| 53 | 40 | 8 | 92 |
| 65 | 40 | 8 | 92 |

The filtrate was injected and purified, the fractions were collected, concentrated and freeze-dried to obtain the following peptides with a purity of more than 95%:
7, 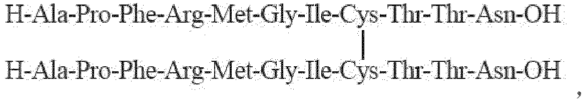 namely peptide (1);
8, 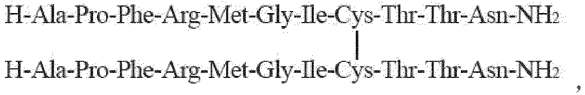 namely peptide (12);
9, 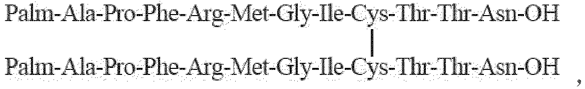 namely peptide (13);
10, 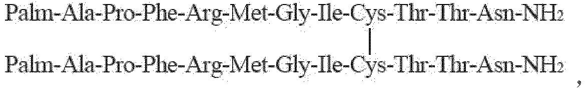 namely peptide (14);
11, 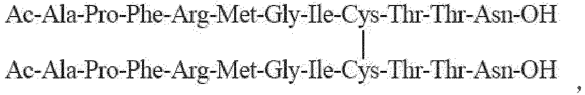 namely peptide (15);
12, 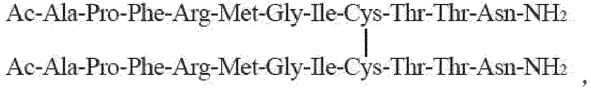 namely peptide (16) .

### Example 10

The side chain mercapto group of Cys8 of the peptide obtained in Example 7 was allyl-modified.

Take 120 mg of the peptide obtained in Example 7, dissolve it in 40 ml of water, protect it under nitrogen, add 5 times the amount of allyl bromide, react at room temperature, monitor the reaction process by HPLC until the reaction is complete, and obtain the following crude peptide:
13, 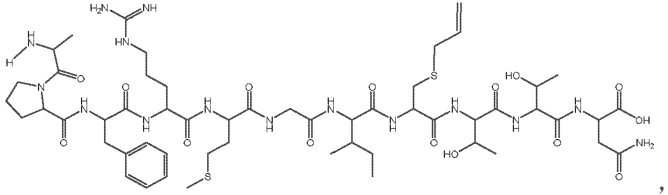 namely peptide (3);
14, 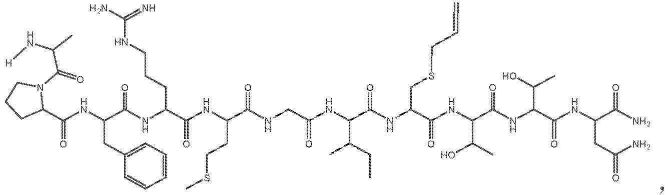 namely peptide (17);
15, 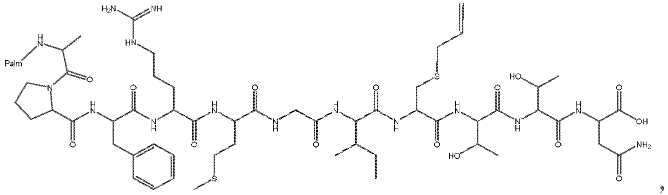 namely peptide (18);
16, 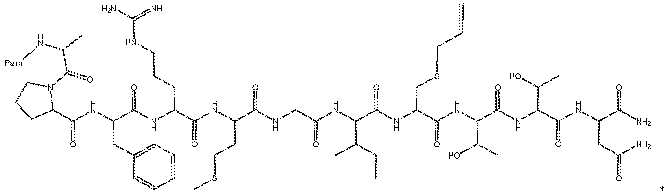 namely peptide (19);
17, 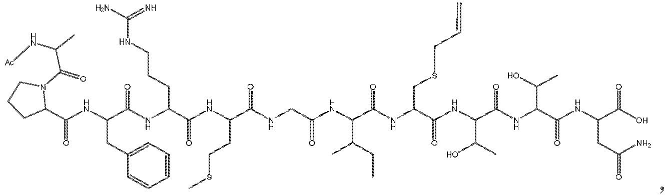 namely peptide (20);
18, 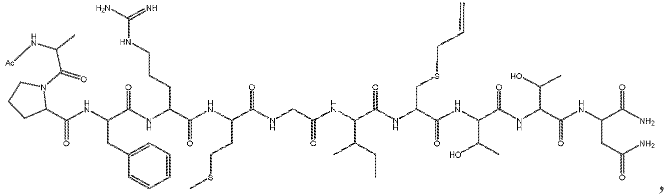 namely peptide (21) .

### Example 11

The side chain thiol of Cys8 of the peptide obtained in Example 7 was modified with tert-butyl bromoacetate.

Take 120 mg of the peptide obtained in Example 7, dissolve it in 40 ml of water, protect it under nitrogen, add 5 times amount of tert-butyl bromoacetate, react at room temperature, monitor the reaction process by HPLC until the reaction is complete, and obtain the following crude peptide:
19, 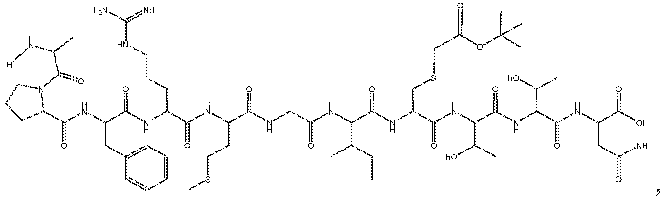 namely peptide (4);
20, 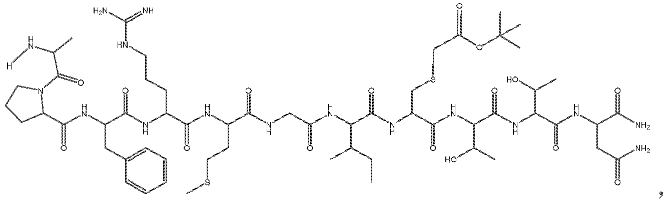 namely peptide (22);
21, 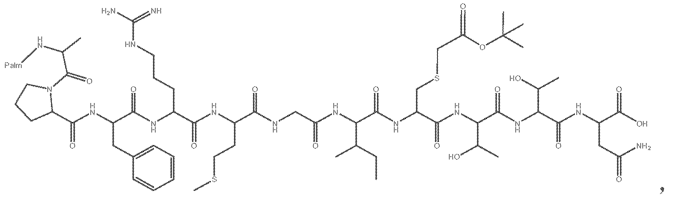 namely peptide (23);
22, 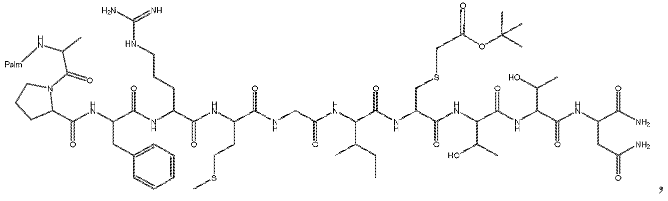 namely peptide (24);
23, 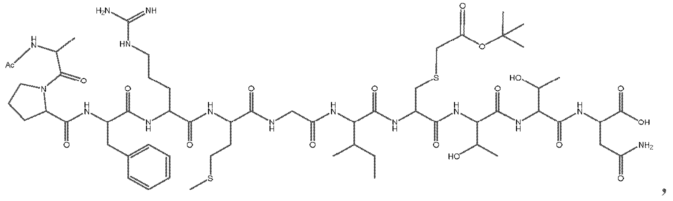 namely peptide (25);
24, 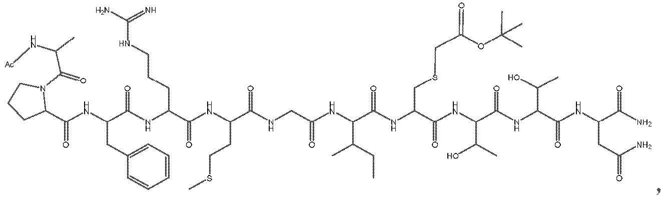 namely peptide (26) .

### Example 12

The mercapto group of the side chain of Cys8 of the peptide obtained in Example 7 was modified with mercaptoethane.

Weigh 120 mg of the peptide obtained in Example 7, dissolve it in 40 ml of water, protect it under nitrogen, add 5 times amount of mercaptoethane, react at room temperature, monitor the reaction process by HPLC until the reaction is complete, and obtain the following crude peptide:
25, 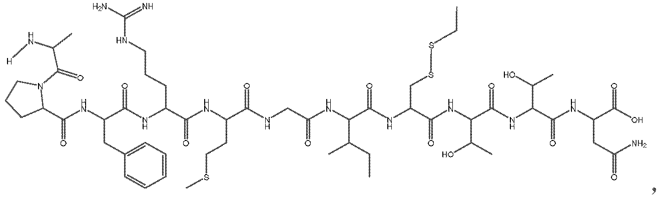 namely peptide (5);
26, 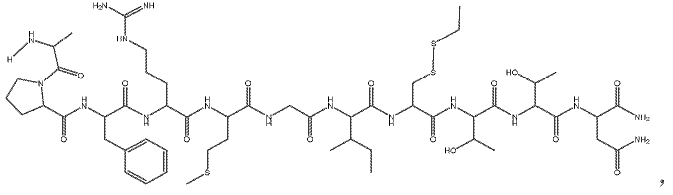 namely peptide (27) ;
27, 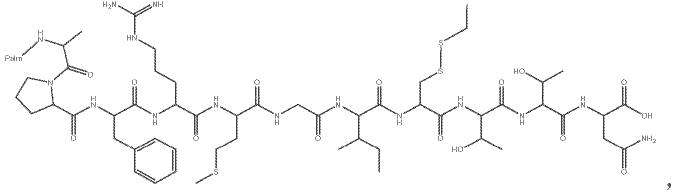 namely peptide (28);
28, 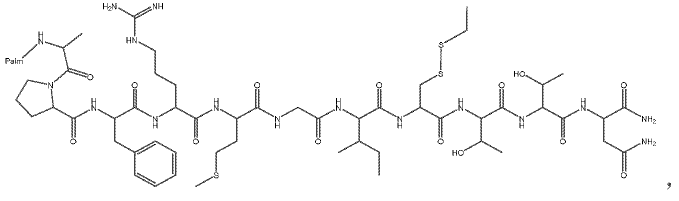 namely peptide (29);
29, 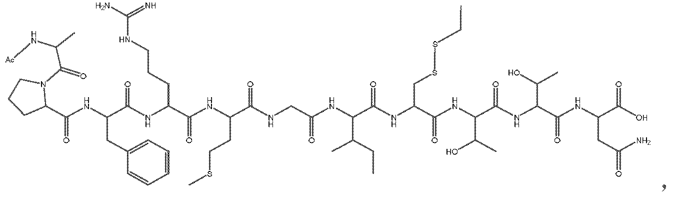 , namely peptide (30);
30, 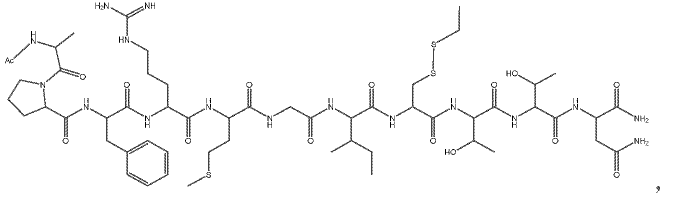 namely peptide (31) .

### Example 13

The thiol group of the side chain of Cys8 of the peptide obtained in Example 7 was modified with hexanethiol.

Weigh 120 mg of the peptide obtained in Example 7, dissolve it in 40 ml of water, protect it under nitrogen, add 5 times amount of hexanethiol, react at room temperature, monitor the reaction process by HPLC until the reaction is complete, and obtain the following crude peptide:
31, 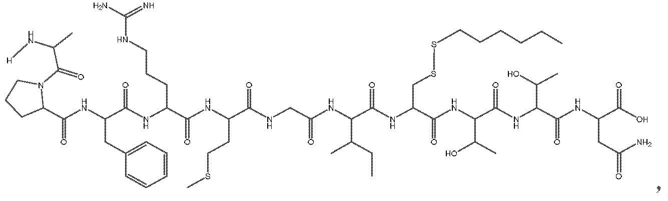 namely peptide (6);
32, 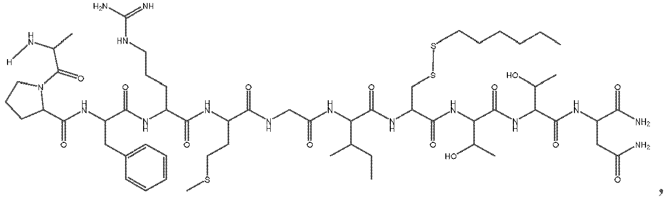 namely peptide (32);
33, 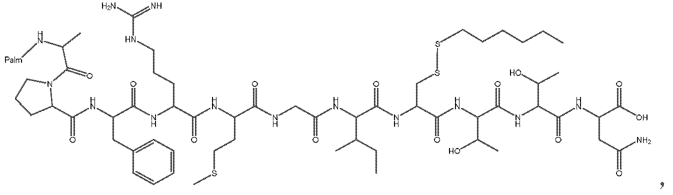 namely peptide (33);
34, 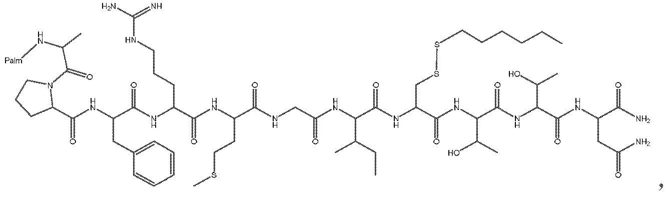 namely peptide (34);
35, 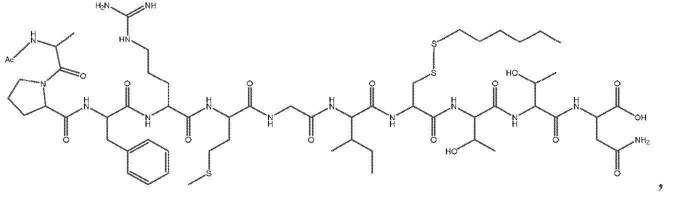 namely peptide (35) ;
36, 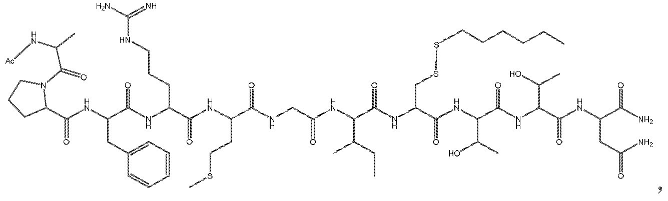 namely peptide (36) .

### Example 14

The side chain sulfhydryl group of Cys8 of the peptide obtained in Example 7 was modified with bromoacetic acid.

Weigh 120 mg of the peptide obtained in Example 7, dissolve it in 40 ml of water, protect it under nitrogen, add 5 times amount of bromoacetic acid, react at room temperature, monitor the reaction process by HPLC until the reaction is complete, and obtain the following crude peptide:
37, 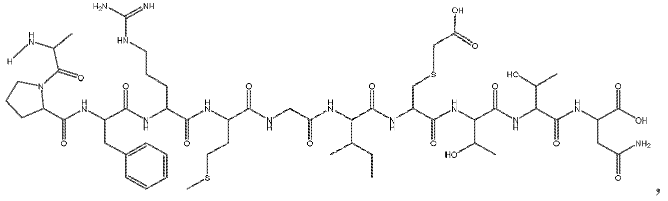 , namely peptide (7);
38, 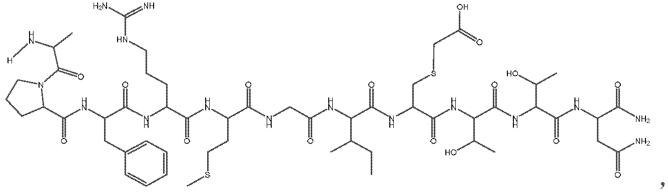 namely peptide (37);
39, 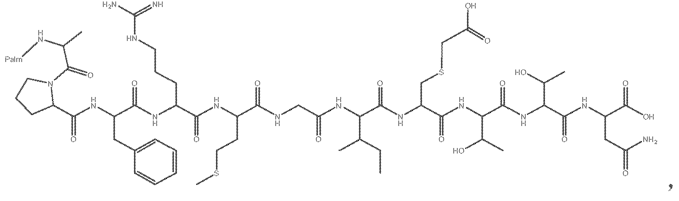 namely peptide (38);
40, 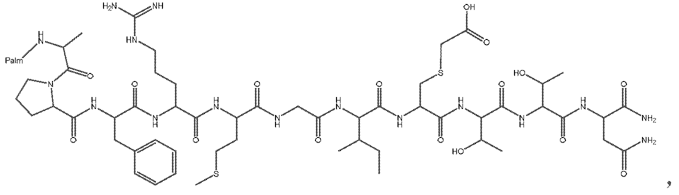 namely peptide (39);
41, 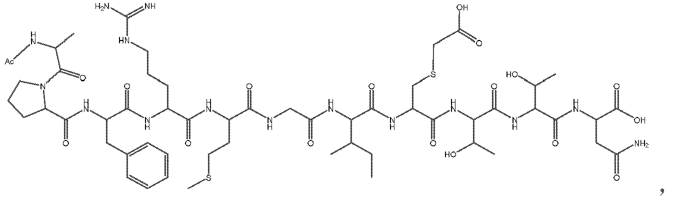 namely peptide (40);
42, 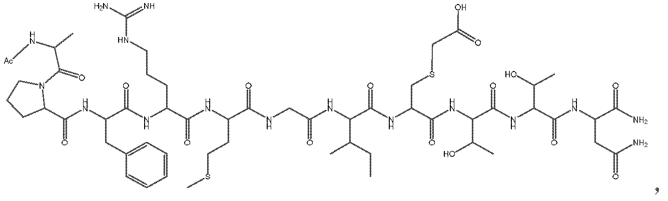 namely peptide (41) .

### Example 15

The products obtained in Examples 10-14 were purified by high performance liquid chromatography.

Purification preparation conditions:
Mobile phase A: pure acetonitrile; mobile phase B: 0.1% acetic acid aqueous solution;
Detection wavelength: 215nm;
Chromatographic column: C18-20*150mm;

Elution gradient:

| Time (min) | Flow rate (ml/min) | A% | B% |
|---|---|---|---|
| 0 | 40 | 11 | 89 |
| 48 | 40 | 35 | 65 |
| 56 | 40 | 38 | 62 |
| 59 | 40 | 48 | 52 |
| 60 | 40 | 80 | 20 |
| 70 | 40 | 80 | 20 |

Purification by injection, fractions were collected, concentrated and freeze-dried to obtain a peptide with a purity of more than 80%.

### Example 16

Preparation of Fmoc-Ala-Pro-Phe-Arg(Pbf)-Met-Gly-Ile-Met-Thr(tBu)-Thr(tBu)-Asn(Trt)-Wang Resin.

### 16.1 Resin Swelling

Weigh 35g (49.7mmol) of Wang Resin into a 2L solid-phase synthesis reaction column. Add 100ml of DMF to completely immerse the resin, then enter nitrogen gas, turn on the nitrogen gas stiring and blowing for 5 minutes, and then drain the solvent for 5 minutes. Continue to add 100ml of DMF, completely immerse the resin, and enter nitrogen gas for 30 minutes, the resin is completely swollen, and the solvent is drained. Then add 100ml of DMF, completely immerse the resin and then enter nitrogen gas, turn on the nitrogen gas stiring and blowing, drain the solvent for 5 minutes, and the swelling is over.

### 16.2 Feeding reaction

Weigh 53.38g of Fmoc-Asn(Trt)-OH (89.46mmol; 1.8 equiv), and add 14.51g of HOBt into a dry 500ml round-bottomed ground flask. Add DMF to dissolve it, and put it in a refrigerator at ±5°C for 10 minutes. Add 20.78ml DIC to activate for 10min, avoid water vapor. The activated amino acid was added to the resin to react for 2 hours, the reaction temperature was controlled at 25° C to 35° C, and the reaction liquid was removed. Wash the resin with DMF, 100ml each time, mix the resin and solvent evenly, and then stir for 2min by timing, and drain the solvent. The resin was washed several times with DMF until the solvent filtered out with suction was clear and transparent.

The degree of substitution of the resin was detected to be 0.5, and the calculated synthesis scale was 24.9 mmol.

The N-terminal Fmoc group was deprotected, and 24.74 g of activated Fmoc-Thr(tBu)-OH (62.25 mmol; 2.5 eq.) was coupled onto the peptidyl resin using DMF as solvent in the presence of 10.09 g HOBt and 14.46 ml DIC, and the reaction was continued for 2h. The resins were then washed and the deprotection treatment of the Fmoc group was repeated for coupling of the next amino acid. In each coupling, 24.74 g of Fmoc-Thr(tBu)-OH (62.25 mmol; 2.5 equiv); 23.12 g of Fmoc-Met-OH (62.25 mmol; 2.5 equiv); 22 g of Fmoc-Ile-OH (62.25 mmol; 2.5 equiv); 18.51 g of Fmoc-Gly-OH (62.25 mmol; 2.5 equiv) were coupled sequentially using DMF as solvent in the presence of 10.09 g HOBt and 14.46 ml DIC.

The N-terminal Fmoc group was deprotected, 32.37 g of activated Fmoc-Met-OH (87.15 mmol; 3.5 equiv) was coupled onto the peptidyl resin using DMF as solvent in the presence of 14.13 g HOBt and 20.24 ml DIC, the reaction was continued for 2h. The resins were then washed and the deprotection treatment of the Fmoc group was repeated for coupling of the next amino acid. In each coupling, 56.54 g of Fmoc-Arg(Pbf)-OH (87.15 mmol; 3.5 equiv); 33.76 g ofFmoc-Phe-OH (87.15 mmol; 3.5 equiv); 29.40 g of Fmoc-Pro-OH (87.15 mmol; 3.5 equiv) and then 27.13 g of Fmoc-Ala-OH (87.15 mmol; 3.5 equiv) were coupled sequentially using DMF as solvent in the presence of 14.13 g HOBt and 20.24 ml DIC.

After the synthesis, the resin was washed with DMF, 100 ml each time, the resin and the solvent were uniformly mixed, stirred for 2 minutes, and the solvent was drained. The resin was washed several times with DMF until the solvent filtered out with suction was clear and transparent. The process of De Fmoc; De-Fmoc twice with 500ml 20% piperidine/DMF, the first reaction was 5 minutes, the second reaction was 8 minutes, and the solvent was drained for 3 minutes. Wash the resin 7-8 times with DMF, and remove the solvent.

### Example 17

Preparation of Fmoc-Ala-Pro-Phe-Arg(Pbf)-Met-Gly-Ile-Met-Thr(tBu)-Thr(tBu)-Asn(Trt)-Linker-Amide Resin.

### 17.1 Preparation of Fmoc-Linker-Amide Resin

Weigh 50 g (60 mmol) of Amide Resin resin into a 2L solid-phase synthesis reaction column. Wash the resin once with 400ml DMF, swell the resin with 500ml DCM for 20min, and remove the solvent. Wash the resin 3 times with 400ml DMF.

Weigh 80.9g (150mmol) of Fmoc-linker and 24.3g (180mmol) of HOBt into a dry 500ml wide-mouth Erlenmeyer flask. Add about 150ml of DMF solvent to dissolve and place in an ice-water bath to cool for 2 minutes, add DIC30ml (195mmol) to activate for 3 minutes, avoiding water vapor. The activated Fmoc-linker was added to the swollen resin to react for 2 hours, and the reaction solution was removed. Wash the resin 3 times with 400ml DMF, 2min each time. After adding the mixture of 28ml (300mmol) of Ac₂O, 10.7ml (60mmol) of DIPEA and 400ml of DMF to seal for 3 hours, the resin was washed four times with 500ml of DMF and twice with 500ml of DCM. Remove the solvent.

### 17.2 De-Fmoc

Fmoc-Linker-Amide Resin was de-Fmoc twice with 500ml of 20% piperidine/DMF, the first reaction was 5 minutes, the second reaction was 8 minutes, and the solvent was drained for 3 minutes. Wash the resin 4 times with 500ml DMF, wash the resin twice with 500ml DCM. Remove the solvent.

### 17.3 Feeding reaction

Weigh 52.21 g of Fmoc-Asn(Trt)-OH (87.5 mmol), and add 14 g of HOBt into a dry 500 ml wide-mouth Erlenmeyer flask. Add DMF solvent to dissolve and place in an ice-water bath to cool. Add 20ml DIC to activate for 3min, avoid water vapor. The activated amino acid was added to the deprotected resin to react for 2 hours, and the reaction solution was removed. Wash the resin twice with 500ml DMF, 2min each time, wash the resin twice with 400ml DCM, and remove the solvent.

The N-terminal Fmoc group was deprotected, 34.78 g of activated Fmoc-Thr(tBu)-OH (87.5 mmol) was coupled onto the peptidyl resin using DMF as solvent in the presence of 14 g HOBt and 20 ml DIC, the reaction was continued for 2h. The resins were then washed and the deprotection treatment of the Fmoc group was repeated for coupling of the next amino acid. In each coupling, 34.78 g of Fmoc-Thr(tBu)-OH (87.5 mmol); 32.50 g of Fmoc-Met-OH (87.5 mmol); 30.92 g of Fmoc-Ile-OH (87.5 mmol); 26.01 g of Fmoc-Gly-OH (87.5 mmol); 32.50 g of Fmoc-Met-OH (87.5 mmol); 56.77 g of Fmoc-Arg (Pbf)-OH (87.5 mmol); 33.90 g of Fmoc-Phe-OH (87.5 mmol); 29.52 g of Fmoc-Pro-OH (87.5 mmol) followed by 27.24 g of Fmoc-Ala-OH (87.5 mmol) were coupled sequentially using DMF as solvent in the presence of 14 g HOBt and 20 ml DIC.

After this synthesis, the resin was washed 4 times with 600 ml DMF for 2 min each, 2 times with 500 ml DCM and the solvent was removed.

### Example 18

The N-terminal Fmoc protecting group was removed.

By deprotecting the N-terminal Fmoc group of these peptidyl resins obtained in Example 16 and Example 17, de-Fmoc twice with 500ml of 20% piperidine/DMF, the first reaction 5min, the second reaction 8min, drain the solvent for 3 min. Wash the resin 4 times with 600ml DMF, 2min each time, wash the resin 2 times with 500ml DCM, and remove the solvent.

### Example 19

The R₁ palmitoyl group was introduced onto the peptidyl resin obtained in Example 18.

In the presence of 1.53 g of HOBt (10 mmol; 10 equiv) and 1.54 mL of DIC(10 mmol; 10 equiv), 2.56 g of palmitic acid (10 mmol; 10 equiv) pre-dissolved in 1 ml of DMF was added to 1 mmol of the peptidyl resin obtained in Example 18. They were allowed to react for 15 h, after which the resin was washed 4 times with 600 ml DMF for 2 min each, 2 times with 500 ml DCM, and the solvent was removed.

### Example 20

The R₁ acetyl group was introduced onto the peptidyl resin obtained in Example 18. 1 mmol of the peptidyl resin obtained in Example 18 was treated with 25 equiv of Ac₂O using 5 mL of DMF as solvent in the presence of 25 equiv of DIPEA. They were allowed to react for 30 min, after which the resin was washed 4 times with 600 ml DMF for 2 min each, 2 times with 500 ml DCM, and the solvent was removed.

### Example 21

The peptidyl resins obtained in Examples 18, 19 and 20 were cleaved from the polymer support. The peptidyl resins obtained in Examples 18, 19 and 20 were shrunk three times with 500 ml, 300 ml, and 200 ml of methanol. Vacuum dry.

Weigh 1 g of the obtained dried peptidyl resins of Examples 18, 19 and 20, and treat with 7 ml of lysate (TFA: EDT: anisole: thioanisole: TIS: H₂O=87.5:2.5:2.5:2.5:2.5:2.5) for 2.5 hours. Suction filter with a sand core funnel, wash the resin with 1ml of trifluoroacetic acid, slowly add the filtrate dropwise to 64ml of frozen isopropyl ether, a large amount of white solid precipitates, wash with isopropyl ether for 6 times after centrifugation. The final precipitate was dried under vacuum.

### Example 22

Purification was carried out by high performance liquid chromatography.

The crude peptide obtained in Example 21 was dissolved with methanol and water, and filtered through a filter membrane with a pore size of 0.45 µm.

Purification preparation conditions:
Mobile phase A: pure acetonitrile; mobile phase B: 0.1% acetic acid aqueous solution;
Detection wavelength: 215nm;
Chromatographic column: C18-20*150mm;

Elution gradient:

| Time(min) | Flow rate (ml/min) | A% | B% |
|---|---|---|---|
| 0 | 40 | 10 | 90 |
| 4 | 40 | 12 | 88 |
| 64 | 40 | 32 | 68 |
| 65 | 40 | 10 | 90 |
| 75 | 40 | 10 | 90 |

The filtered sample was injected and purified, the fractions were collected, concentrated and freeze-dried to obtain the following peptides with a purity of over 90%:
43. H-Ala-Pro-Phe-Arg-Met-Gly-Ile-Met-Thr-Thr-Asn-OH, namely peptide (2);
44. H-Ala-Pro-Phe-Arg-Met-Gly-Ile-Met-Thr-Thr-Asn-NH₂, namely peptide (42);
45. Palm-Ala-Pro-Phe-Arg-Met-Gly-Ile-Met-Thr-Thr-Asn-OH, namely peptide (43);
46. Palm-Ala-Pro-Phe-Arg-Met-Gly-Ile-Met-Thr-Thr-Asn-NH₂, namely peptide (44);
47. Ac-Ala-Pro-Phe-Arg-Met-Gly-Ile-Met-Thr-Thr-Asn-OH, namely peptide (45);
48. Ac-Ala-Pro-Phe-Arg-Met-Gly-Ile-Met-Thr-Thr-Asn-NH₂, namely peptide (46).

### Example 23

Other compounds of the general formula (I) of the present invention can be prepared by similar methods.

The molecular weights of the obtained peptides were determined by ESI-MS, and the test results of some compounds were shown in Table 2 below.

**Table 2. Determination of molecular weight by mass spectrometry**

| No. | Sequence/Structure | Molecular Weight By Mass Spectrometry |
|---|---|---|
| (1) | 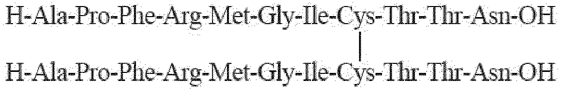 | 2418.45 |
| (2) | H-Ala-Pro-Phe-Arg-Met-Gly-Ile-Met-Thr-Thr-Asn-OH | 1237.58 |
| (3) | 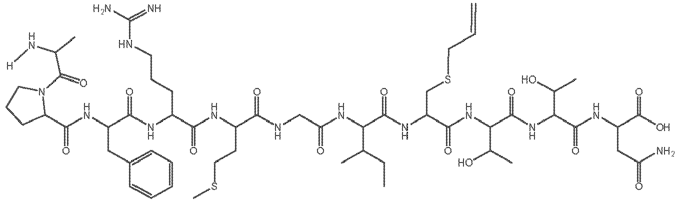 | 1249.57 |
| (4) | 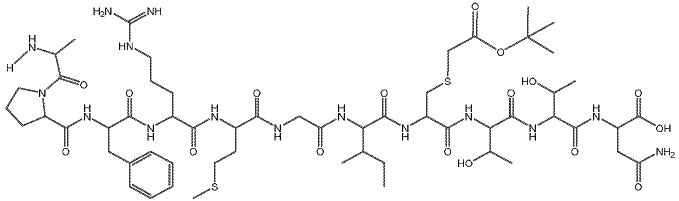 | 1323.61 |
| (5) | 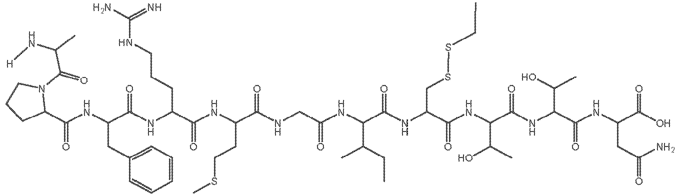 | 1269.55 |
| (6) | 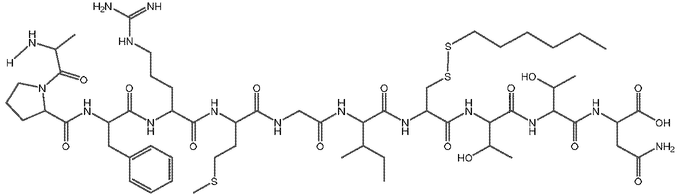 | 1325.60 |
| (7) | 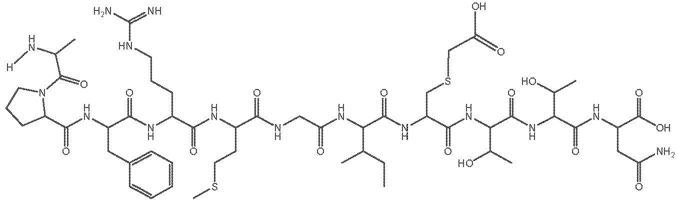 | 1267.54 |

### Example 24 Cell Proliferation Experiment

### 24.1 Reagents and materials

Phosphate buffered saline (PBS) (Gibco), thiazolium blue (MTT) (Sigma), dimethylsulfoxide (DMSO) (Sigma), high glucose medium (DMEM) (Gibco), fetal bovine serum (Gibco).

### 24.2 Instruments

Microplate reader (MD, USA), CO₂ incubator (Shanghai Yiheng), ultra-clean bench (Suzhou Purification), biological inverted microscope (Chongqing Optoelectronics).

### 24.3 Cell lines

Human keratinocytes (HaCaT) were purchased from the Kunming Cell Bank of the Type Culture Collection Committee of the Chinese Academy of Sciences, and mouse skin fibroblasts (NIH3T3) were purchased from the Shanghai Cell Bank of the Type Culture Collection Committee of the Chinese Academy of Sciences.

### 24.4 Samples to be tested

Administration groups: peptide (1), peptide (2), peptide (3), peptide (4), peptide (5), the concentrations were 12.5ppm, 25ppm, 50ppm, 100ppm respectively.

Control group: PBS blank control.

### 24.5 Experimental methods

a) Take cells in good condition in exponential growth phase, add 0.25% trypsin digestion solution, digest to make adherent cells fall off, count 1-4×10⁵ cells/mL, and make cell suspension;
b) Appropriately dilute 2000 cells/well of the cell suspension and inoculate it on a 96-well plate at 200 µL/well, and culture it in a constant temperature CO₂ incubator for 24 hours.
c) Change the medium, add the sample to be tested, 20 µL/well, and incubate for 24 hours;
d) Add the MTT reagent into the 96-well plate, 20 µL/well, and react in the incubator for 4 hours;
e) Aspirate the supernatant, add DMSO, 150 µL/well, and shake on a plate shaker for 5 minutes;
f) After 5 minutes, use a microplate reader to read the reference OD values at 490nm and 630nm wavelengths, and calculate the cell viability: Cell viability=[OD(administration well)-OD(zero adjustment well)]/[OD(control well)-OD(zero adjustment well)]×100%

### 24.6 Results

According to the principle of parallel design, the experiments were repeated at least three times, and the experimental data were expressed as mean ± SD, and SPSS 17.0 statistical software was used for data analysis.

MTT method is a method to detect cell survival and growth. The measured OD value is directly proportional to cell activity. The larger the OD value, the higher the cell activity.

See Figure 1 for the effects of peptides (1) to (5) on the activity of HaCaT cells. The results showed that peptide (1)-peptide (5) could all improve the activity of HaCaT cells to a certain extent, and peptide (2) and peptide (3) had a greater impact on the activity of HaCaT cells. Peptide (2) or peptide (3) of 12.5ppm-100ppm could both improve the activity of HaCaT cells, promote their proliferation.

See Figure 2 for the effects of peptides (1) to (5) on the activity of NIH3T3 cells. The results showed that peptide (1)-peptide (5) could all improve the activity of NIH3T3 cells to a certain extent, and peptide (3) had a greater impact on the activity of NIH3T3 cells. Peptide (3) could improve the activity of NIH3T3 cells and promote their proliferation at each dose of 12.5ppm-100ppm.

### Example 25 Promoting Cell Adhesion Test

### 25.1 Reagents and materials

Phosphate buffered saline (PBS) (Gibco), thiazolium blue (MTT) (Sigma), dimethylsulfoxide (DMSO) (Sigma), high glucose medium (DMEM) (Gibco), fetal bovine serum (Gibco).

### 25.2 Instruments

Microplate reader (MD, USA), CO₂ incubator (Shanghai Yiheng), ultra-clean bench (Suzhou Purification), biological inverted microscope (Chongqing Optoelectronics).

### 25.3 Cell lines

Human keratinocytes (HaCaT) were purchased from the Kunming Cell Bank of the Type Culture Collection Committee of the Chinese Academy of Sciences.

### 25.4 Samples to be tested

Administration group: peptide (1), peptide (2), the concentrations were 12.5ppm, 25ppm, 50ppm, 100ppm respectively. Dissolve with PBS solution.

Control group: PBS blank control.

### 25.5 Experimental methods

The cryopreserved HaCaT human cuticle cells were cultured, subcultured at a ratio of 1:2 to about 5 passages, and the cells with better growth were selected as the experimental objects.

The sample to be tested was added to a 96-well plate at a rate of 20 µL/well, and dried overnight in a constant temperature oven at 37°C. On the second day, after digesting the well-growing HaCaT cells, seed them to the plate at a density of 10,000 HaCaT cells/well, supplement the medium to 200 µL, and incubate for 3 hours at 37°C and 5% CO₂ incubator. After the culture is over, take out the culture plate and continue to replenish the culture medium until the liquid level just overflows, and seal it with a parafilm to ensure that there were no air bubbles. Turn over for 20 minutes under the action of a three-dimensional force. The original medium was discarded, and 90 µL of fresh medium and 10 µL of 5 mg/mL MTT were added to each well, and placed in a 37° C., 5% CO₂ incubator for 3 h. Discard the solution and add 150 µL of DMSO. Set the detection wavelength to 490nm, the reference wavelength to 630nm, and use a microplate reader to read the OD value.

### 25.6 Results

By selecting HaCaT cells, plate them in a drug-coated 96-well plate, and after incubation and a period of three-dimensional force, the cells with strong adhesion can be maintained on the 96-well plate. By performing MTT quantitative analysis on the live cells on the plate, that is, the effect of the drug on cell adhesion can be evaluated to determine whether the peptide of the present invention can improve cell elasticity. After three-dimensional force action, the more living cells remained on the 96-well plate, the greater the measured OD value, indicating the stronger the adhesion of the cells.

The experimental results were shown in Figure 3. Compared with the normal control group, both peptide (1) and peptide (2) could improve the adhesion ability of HaCaT cells, which was beneficial to improve their elasticity.

### Example 26 Scratch Test

### 26.1 Reagents and materials

Phosphate buffered saline (PBS) (Gibco), high glucose medium (DMEM) (Gibco), fetal bovine serum (Gibco).

### 26.2 Instruments

Light microscope, CO₂ incubator.

### 26.3 Cell lines

Human keratinocytes (HaCaT) were purchased from the Kunming Cell Bank of the Type Culture Collection Committee of the Chinese Academy of Sciences.

### 26.4 Samples to be tested

Administration group: 100 ppm of peptide (1), 100 ppm of peptide (2), 50 U/mL of epidermal growth factor (EGF).

Control group: PBS blank control.

### 26.5 Experimental methods

The cryopreserved HaCaT cells were cultured, subcultured to about 5 passages according to the ratio of 1:2, and the cells with better growth were selected as the experimental objects. Cells were seeded in a 12-well plate at a density of 200,000 cells/well, with 2 mL of cell suspension in each well. After the cells adhered to the wall, the medium was replaced with 0.5% to 1% fetal bovine serum to maintain the cells for 24 hours to synchronize them. change. Scratch with a sterilized pipette tip, wash off the dropped cells with PBS, add the sample to be tested, continue to incubate in a 37°C, 5% CO₂ incubator, and place it under an optical microscope after 24 hours to analyze the wound healing area.

### 26.6 Results

The cell scratch migration test can reflect the proliferation and repair ability of the cells. The results were shown in Figure 4. The cell scratches in the normal control group were still obvious, and the scratch spacing was normal, compared with the control group, the cell proliferation and migration in the EGF positive group was obvious, the spacing was significantly shortened; while the scratch spacing of peptide (1) and peptide (2) was shorter than that of the normal control group, and cell migration tracks appeared. The results proved that the peptide of the present invention could promote proliferation and migration of HaCaT human keratinocytes , had the effect of skin repair.

### Example 27 Light Damage Repair Experiment

### 27.1 Reagents and materials

Phosphate buffered saline (PBS) (Gibco), thiazolium blue (MTT) (Sigma), dimethylsulfoxide (DMSO) (Sigma), high glucose medium (DMEM) (Gibco), fetal bovine serum (Gibco).

### 27.2 Instruments

Microplate reader (MD, USA), CO₂ incubator (Shanghai Yiheng), ultra-clean bench (Suzhou Purification), biological inverted microscope (Chongqing Optoelectronics).

### 27.3 Cell lines

Human keratinocytes (HaCaT) were purchased from the Kunming Cell Bank of the Type Culture Collection Committee of the Chinese Academy of Sciences, and mouse skin tibroblasts (NIH3T3) were purchased from the Shanghai Cell Bank of the Type Culture Collection Committee of the Chinese Academy of Sciences.

### 27.4 Samples to be tested

The concentrations of peptide (1), peptide (2), peptide (3), peptide (4), and peptide (5) were 12.5 ppm, 25 ppm, 50 ppm, and 100 ppm, respectively.

### 27.5 Experimental methods

Take cells in good exponential growth phase, add 0.25% trypsin digestion solution, digest to make adherent cells fall off, count 1×10⁵-4×10⁵ cells/mL, and make cell suspension.

Cells were seeded into 96-well plates at 10,000 cells/well, and the UVB photodamage model was established when the cells reached about 80% confluent. Add 50 µL of medium without UVB irradiation, and use it as a blank control group; add appropriate amount of PBS to the experimental group and wash repeatedly until colorless, then add 50 µL of PBS, and irradiate with 80J/cm³ UVB lamp, the distance between the lamp source and the culture bottle is 15cm, after the experimental group was irradiated, the PBS was discarded, the culture medium and the doubly diluted drug were added to 200 µL, and the incubation was continued for 24 hours at 37°C and 5% CO₂ incubator.

After 24 hours, add 22 µL of 5 mg/mL MTT to each well, place in an incubator and incubate for 4 hours, discard the original solution, and add 150 µL/well of DMSO. After 5 minutes, use a microplate reader to read the reference OD values at 490nm and 630nm wavelengths.

### 27.6 Results

By establishing a UVB light-damaged cell model, administering 24 hours after ultraviolet light irradiation, and using the MTT method to detect cell proliferation activity, the ability of the peptide of the present invention to repair light-damaged cells was evaluated.

The results of photodamage repair of HaCaT cells were shown in Figure 5. The results showed that compared with the non-irradiated normal control group, the cell viability was significantly reduced after UVB irradiation, suggesting that the light-damaged model was successfully modeled. After administration, peptide (1)-peptide (5) could all improve the activity of photodamaged HaCaT cells to a certain extent, and peptides (2) and (3) had a better effect on improving cell activity. The results proved that the peptide of the present invention could repair HaCaT cells damaged by light, improve their activity, and had good skin repair ability.

The results of photodamage repair of NIH3T3 cells were shown in Figure 6. The results showed that compared with the non-irradiated normal group, the activity of NIH3T3 cells after irradiation was significantly reduced, suggesting that the modeling of photodamage model was successful. After administration, peptide (1) can significantly increase cell activity at 50 and 100ppm; peptide (2) can significantly increase NIH3T3 cell activity at each dose of 12.5ppm-100ppm. The results proved that both peptide (1) and peptide (2) could repair photodamaged NIH3T3 cells within a certain concentration range, improve their activity, and had good skin repair ability.

### Example 28 Anti-Inflammatory Repair Experiment

### 28.1 Reagents and materials

LPS, dexamethasone (DXM), ELISA kit.

### 28.2 Instruments

Microplate reader (MD, USA), CO₂ incubator (Shanghai Yiheng), ultra-clean bench (Suzhou Purification).

### 28.3 Cell lines

Mouse mononuclear macrophages (RAW264.7 cells).

### 28.4 Samples to be tested

Peptide (2), tested at a concentration of 100 ppm.

### 28.5 Experimental methods

a) Take RAW264.7 cells in good exponential growth phase, add 0.25% trypsin digestion solution, digest to make adherent cells fall off, count 1-4×10 ⁶ cells/mL, and make cell suspension;
b) Appropriately dilute 100,000 cells/well of cell suspension and inoculate it on a 6-well plate, and establish an LPS-stimulated inflammation model when the cells reached about 80% confluent. Set blank control group (control), LPS group, dexamethasone group (DXM), polypeptide group. Add 1000 µL culture medium to the blank control group; add 200 ng/mL LPS to the LPS group, add culture medium to 1000 µL; add 200 ng/mL LPS and 50 ng/mL dexamethasone to the dexamethasone group, add culture medium to 1000 µL; add 200ng/mL LPS and peptide (2) to the polypeptide group, add culture medium to 1000µL (final test concentration of peptide is 100ppm). Continue to incubate at 37°C, 5% CO₂ incubator for 48h;
c) Centrifuge at 3000rmp for 10min to collect the cell supernatant to obtain the sample liquid;
d) Operate according to the ELISA operating instructions: move all reagents to room temperature and equilibrate for at least 30 minutes, prepare reagents according to the instructions, and set aside. Set up wells for standard products and wells for samples to be tested, add 100 µL of standard products or samples to be tested in each well, shake gently, cover with plate stickers, and incubate at 37°C for 2 hours. 4 replicate wells were set up for each sample. Discard the liquid, spin dry, and wash the plate 5 times. Add 100 µL of biotin-labeled antibody working solution to each well, cover with a new plate sticker, and incubate at 37°C for 1 h. Discard the liquid in the well, spin dry, wash the plate 5 times, soak for 1 min each time, 200 µL/well, and spin dry. Add 100 µL of horseradish peroxidase-labeled avidin working solution to each well, cover with a new plate, and incubate at 37°C for 1 h. Discard the liquid in the well, spin dry, wash the plate 5 times, soak for 1 min each time, 200 µL/well, and spin dry. Add 100 µL of substrate solution to each well sequentially, and develop color for 15-30 minutes at 37°C in the dark. Add 100 µL of stop solution to each well sequentially to stop the reaction. The OD value of each well was sequentially measured at 450 nm with a microplate reader within 15 min after the reaction was terminated.

### 28.6 Experimental Results

Inflammatory factors such as TNF-α, IL-6 and IL-8 can cause excessive immune response of the body, leading to skin ulceration, redness and swelling, etc. Therefore, inhibiting inflammatory factors such as TNF-α, IL-6 and IL-8 can achieve anti-inflammatory repair effect. The inflammation model was established by stimulating RAW264.7 cells with 200ng/mL LPS, and the anti-inflammatory repairing effect of the peptide of the present invention was explored. The experimental results were shown in Figure 7-9. After LPS stimulation, TNF-α, IL-6 and IL-8 cytokines were significantly up-regulated; after administration, 100ppm of peptide (2) significantly down-regulated the expression of TNF-α, IL-6 and IL-8, its anti-inflammatory effect was equivalent to the positive drug dexamethasone, indicating that peptide (2) had obvious anti-inflammatory repair effect.

### Example 29 Stability Experiment Research

1. Peptide H-Ala-Pro-Phe-Arg-Met-Gly-Ile-Cys-Thr-Thr-Asn-OH (hereinafter referred to as reference peptide A) sample and peptide (1), peptide (2) samples were dissolved in water respectively, placed under normal temperature conditions, and the purity changes of each sample were investigated by HPLC at 0 days, 8 days, and 18 days. The results were shown in the following Table 3:

**Table 3 Stability studies of reference peptide A aqueous solution and peptide (1), peptide (2) aqueous solution**

| Test conditions | Reference peptide A aqueous solution (purity) | Peptide (1) aqueous solution (purity) | Peptide (2) aqueous solution (purity) |
|---|---|---|---|
| Solution, Day 0 | 88.030% | 98.443% | 99.291% |
| Day 8 at room temperature | 80.880% | 98.362% | 99.122% |
| Day 18 at room temperature | 72.693% | 96.703% | 98.045% |

From the results in Table 3, it could be seen that the purity of reference peptide A in aqueous solution decreased significantly at 0 day when it was exposed to the air. And its purity dropped to 72.693% after being placed in the aqueous solution at room temperature for 18 days. Compared with the reference peptide A aqueous solution, the aqueous solutions of peptide (1) and peptide (2) were left at room temperature for 18 days, and their purity did not change significantly.
2. Stability study of reference peptide A powder, peptide (1) powder and peptide (2) powder The reference peptide A powder, peptide (1) powder, and peptide (2) powder were placed at 25°C and 40°C respectively, and the changes of their purity over time were investigated by HPLC. The results were shown in Table 4 below:

**Table 4 Stability studies of reference peptide A powder, peptide (1) powder, and peptide (2) powder**

| Test conditions | Reference peptide A powder (purity) | Test conditions | Peptide (1) powder (purity) | Peptide (2) powder (purity) |
|---|---|---|---|---|
| Powder, Day 0 | 88.030% | Powder, Day 0 | 98.442% | 99.291% |
| 25°C, Day 5 | 85.913% | 25°C, Day 20 | 98.331% | 99.146% |
| 40°C, Day 5 | 57.503% | 40°C, Day 20 | 96.716% | 98.583% |

It could be seen from the results in Table 4 that the purity of the reference peptide A powder was greatly reduced after being placed at 40°C for 5 days, only 57.503%. In contrast, the purity of peptide (1) powder and peptide (2) powder remained unchanged for 20 days at 40°C.
3. Study on the stability of peptide (3)-(7) powder
The powder samples of peptides (3)-(7) were placed under high humidity (RH92.5%, 25°C) conditions for 10 days, and the purity changes of each sample were investigated by HPLC. The results were shown in Table 5 below:

**Table 5 The stability research of peptide (3)-(7) powder**

| Sample serial number | Day 0 (purity) | High humidity, Day 10 (purity) |
|---|---|---|
| Peptide(3) | 97.605% | 96.251% |
| Peptide(4) | 96.017% | 93.962% |
| Peptide(5) | 97.482% | 94.603% |
| Peptide(6) | 96.235% | 95.719% |
| Peptide(7) | 98.541% | 97.863% |

From the results in Table 5, it could be seen that the purity of peptides (3)-(7) did not change significantly when placed under high humidity (RH92.5%, 25°C) for 10 days.

In Example 29, the HPLC chromatographic separation conditions were as follows:
Chromatographic column: Octadecyl bonded silica gel as filler 4.6*250mm, 5µm
Mobile phase A: Acetonitrile (containing 0.09% trifluoroacetic acid)
Mobile phase B: 0.1% trifluoroacetic acid
Diluent: water
Flow rate: 1.0ml/min;
Column temperature: 35°C;
Detection wavelength: 215 nm;
Injection volume: 10 µl;
Running time: 32 min
Gradient elution table:

| Time (min) | Mobile phase A (%) | Mobile phase B (%) |
|---|---|---|
| 0 | 25 | 75 |
| 15 | 45 | 55 |
| 21 | 90 | 10 |
| 22 | 25 | 75 |
| 30 | 25 | 75 |

The test solution: Take an appropriate amount of this product and add water to dissolve and dilute to make a solution containing 1 mg per 1 ml.

### Example 30 Preparation of Liposomes Containing Peptide (1)

| Component | By Weight % |
|---|---|
| Phosphatidylcholine | 4.0 |
| Peptide (1) | 0.2 |
| Preservatives | 0.5 |
| Water | Add to 100 |

Dipalmitoylphosphatidylcholine was weighed out and dissolved in chloroform. The solvent was evaporated under vacuum until a thin layer of phospholipids was obtained, which was hydrated by treatment with an aqueous peptide solution of the desired concentration at 55°C to give multi-lamellar liposomes. The multi-lamellar liposomes were homogenized under high pressure to obtain smaller and uniform single-lamellar liposomes.

### Example 31 Preparation of Microemulsion Composition Containing Peptide (2)

| Component | By Weight % |
|---|---|
| A Sodium diethylhexyl sulfosuccinate | 1.35 |
| Isostearic Acid | 7.65 |
| B Water | 0.2 |
| Denatured Alcohol | 0.8 |
| C Ethylhexyl Cocoate | Add to 100 |
| D peptide (2) | 0.005 |

According to the prescribed dosage, weigh the ingredients of Phase B and add them to the container. Next, add phase D to phase B and homogenize with continuous stirring. Phase A is then added to the mixture. Finally, phase C', is added to obtain a microemulsion composition containing peptide (2).

The above content is a further detailed description of the present invention in conjunction with specific preferred embodiments, but it does not mean that the specific implementation of the present invention is limited to these descriptions. For those of ordinary skill in the field of the present invention, without departing from the concept of the present invention, some simple deduction or substitutions can be made, which should be regarded as belonging to the protection scope of the present invention.

## Claims

1. A peptide represented by formula (I), or a stereoisomer thereof, or a mixture of stereoisomers thereof, or a cosmetically acceptable salt thereof, or a pharmaceutically acceptable salt thereof, 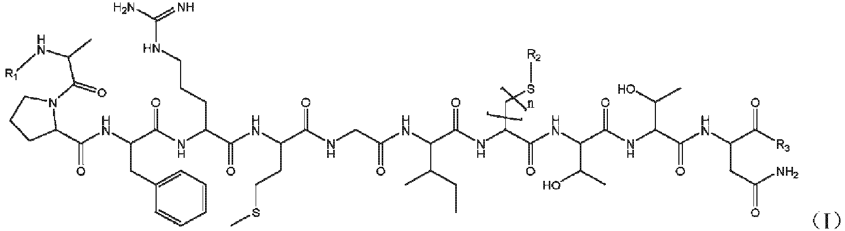 in formula (I),
R₂ is selected from: H, alkyl, alkenylalkyl, carboxyalkyl, esterylalkyl, alkylmercapto, carboxyalkylmercapto, or 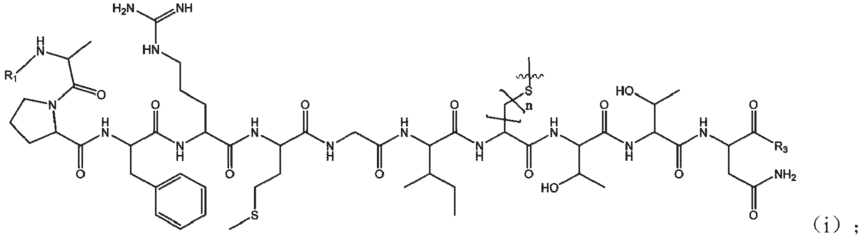 in formula (I) and formula (i),
n is selected from: 1 or 2;
R₁ is selected from: H or R₄-CO-, wherein R₄ is selected from: substituted or unsubstituted alkyl, substituted or unsubstituted alkenyl;
R₃ is selected from: -NR₅R₆ or -OR₅, wherein each R₅ and R₆ are independently selected from:
H, substituted or unsubstituted alkyl, substituted or unsubstituted alkenyl;
the alkyl in the "alkyl", "alkenylalkyl", "carboxyalkyl", "esterylalkyl", "alkylmercapto",
"carboxyalkylmercapto" refers to a saturated aliphatic straight-chain or branched alkyl group having 1-24 carbon atoms (optionally having 1-16 carbon atoms; optionally having 1-14 carbon atoms; optionally having 1-12 carbon atoms; optionally having 1, 2, 3, 4, 5, or 6 carbon atoms);
optionally is selected from: methyl, ethyl, isopropyl, isobutyl, tert-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl in the "alkenyl" and "alkenylalkyl" refers to a straight-chain or branched alkenyl group having 2-24 carbon atoms (optionally having 2-16 carbon atoms; optionally having 2-14 carbon atoms; optionally having 2-12 carbon atoms, optionally having 2, 3, 4, 5, or 6 carbon atoms), said "alkenyl" having one or more carbon-carbon double bonds, optionally have 1, 2 or 3 conjugated or non-conjugated carbon-carbon double bonds; said "alkenyl" is bonded to the rest of the molecule through a single bond; optionally selected from: vinyl, oleyl, or linoleyl;
the esteryl in the "esterylalkyl" is R(C=O)OR' group, wherein R and R' are each independently a single bond, an alkyl group or an alkylene group;
optionally, the substituents in the "substituted alkyl" and "substituted alkenyl" are selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide;
C₁-C₄ alkylsulfonyl; thiol; C₁-C₄ alkylthio; C₆-C₃₀ aryloxy such as phenoxy; - NR_{b}(C=NR_{b})NR_{b}R_{c}, wherein R_{b} and R_{c} are independently selected from: H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, three to ten membered heterocyclic group, or a protecting group for an amino group.

2. The peptide represented by formula (I) according to claim 1, or a stereoisomer thereof, or a mixture of stereoisomers thereof, or a cosmetically acceptable salt thereof, or a pharmaceutically acceptable salt thereof, **characterized in that**, in formula (I), when n is 2, R₂ is selected from: H, alkyl, alkenylalkyl, carboxyalkyl, esterylalkyl, alkylmercapto or carboxyalkylmercapto.

3. The peptide represented by formula (I) according to claim 1 or 2, or a stereoisomer thereof, or a mixture of stereoisomers thereof, or a cosmetically acceptable salt thereof, or a pharmaceutically acceptable salt thereof, **characterized in that**, R₁ is selected from: H, acetyl, tert-butyryl, hexanoyl, 2-methylhexanoyl, octanoyl, caproyl, lauroyl, myristoyl, palmitoyl, stearoyl, oleoyl or linoleoyl;
optionally, R₁ is selected from H, acetyl, lauroyl, myristoyl or palmitoyl;
optionally, R₁ is H, acetyl or palmitoyl.

4. The peptide represented by formula (I) according to claim 1 or 2, or a stereoisomer thereof, or a mixture of stereoisomers thereof, or a cosmetically acceptable salt thereof, or a pharmaceutically acceptable salt thereof, **characterized in that**, R₂ is selected from: H, -CH₃, - CH₂CH₃, -CH₂CH=CH₂, -CH₂COOH, -CH₂COOC(CH₃)₃, -S-CH₂CH₃, -S-(CH₂)₅CH₃, -S-CH₂COOH or 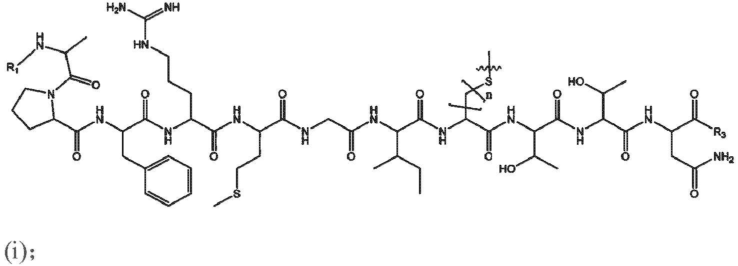 optionally, when n is 2, R₂ is selected from: H, -CH₃, -CH₂CH₃, -CH₂CH=CH₂, -CH₂COOH, - CH₂COOC(CH₃)₃, -S-CH₂CH₃, -S-(CH₂)₅CH₃ or -S-CH₂COOH.

5. The peptide represented by formula (I) according to claim 1 or 2, or a stereoisomer thereof, or a mixture of stereoisomers thereof, or a cosmetically acceptable salt thereof, or a pharmaceutically acceptable salt thereof, **characterized in that**, R₅ and R₆ are independently selected from: H, methyl, ethyl, hexyl, dodecyl or hexadecyl;
optionally, R₅ is H and R₆ is selected from: H, methyl, ethyl, hexyl, dodecyl or hexadecyl;
optionally, R₃ is -OH or -NH₂.

6. The peptide represented by formula (I) according to any one of claims 1-5, or a stereoisomer thereof, or a mixture of stereoisomers thereof, or a cosmetically acceptable salt thereof, or a pharmaceutically acceptable salt thereof **characterized in that**, selected from the following peptides (1)-(46): 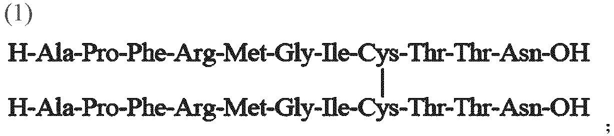
(2) H-Ala-Pro-Phe-Arg-Met-Gly-Ile-Met-Thr-Thr-Asn-OH; 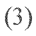 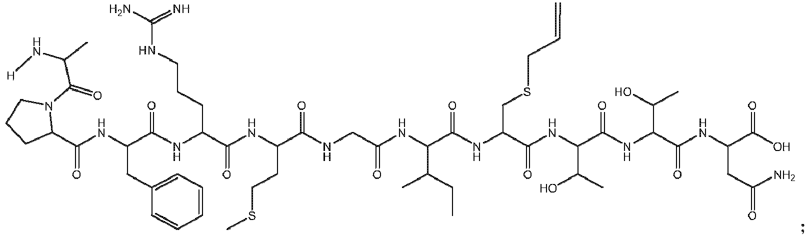 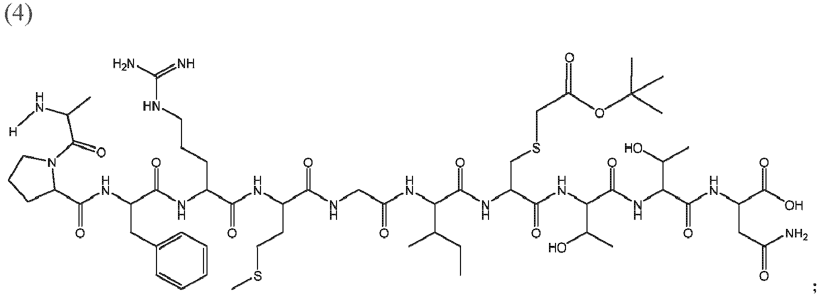 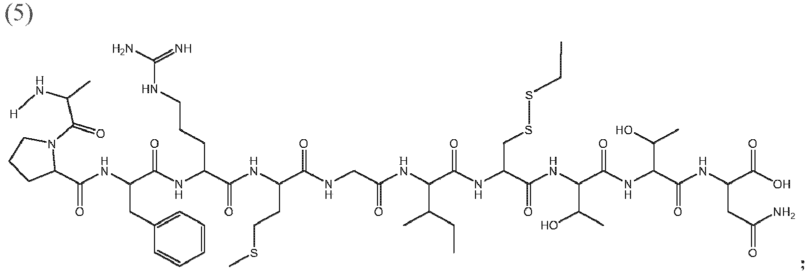 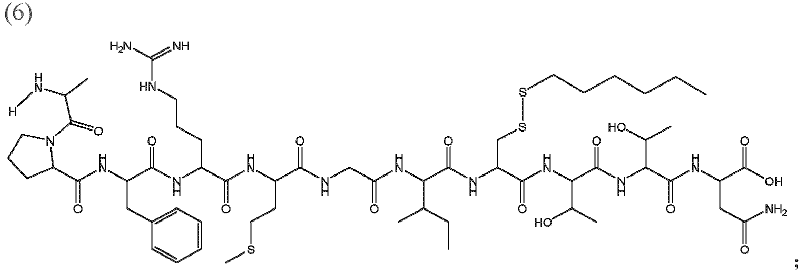 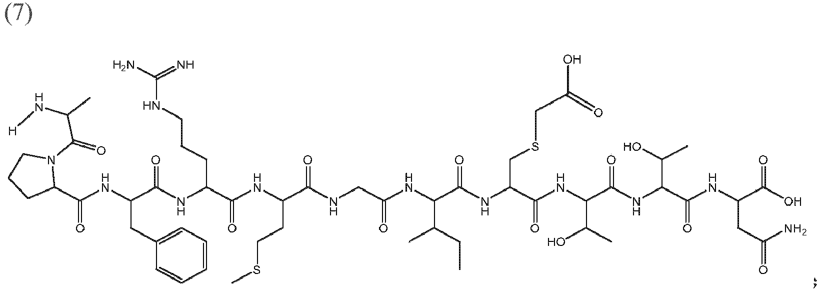
(8) Palm-Ala-Pro-Phe-Arg-Met-Gly-Ile-Cys-Thr-Thr-Asn-OH;
(9) Palm-Ala-Pro-Phe-Arg-Met-Gly-Ile-Cys-Thr-Thr-Asn-NH₂;
(10) Ac-Ala-Pro-Phe-Arg-Met-Gly-Ile-Cys- Thr- Thr-Asn-OH;
(11) Ac-Ala-Pro-Phe-Arg-Met-Gly-Ile-Cys-Thr-Thr-Asn-NBh; 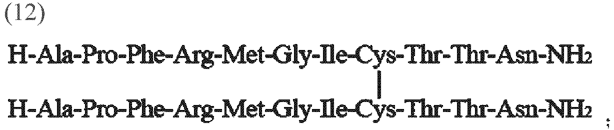 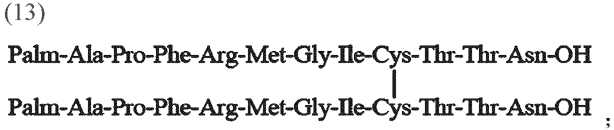 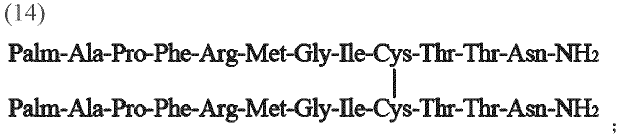 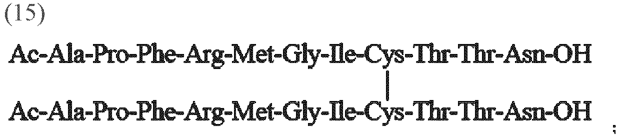 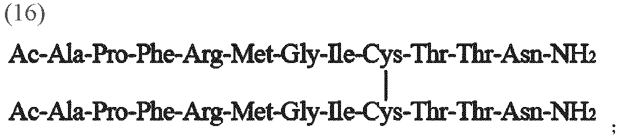 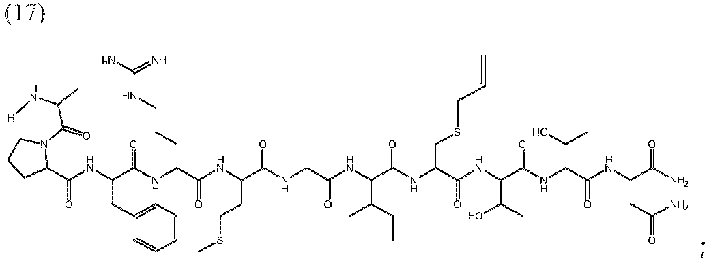 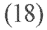 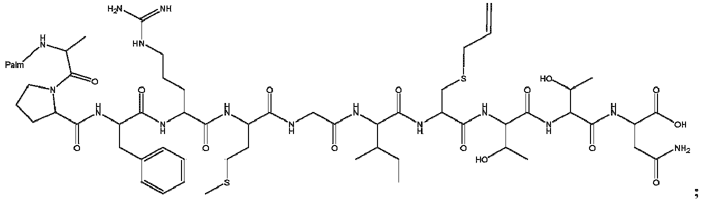 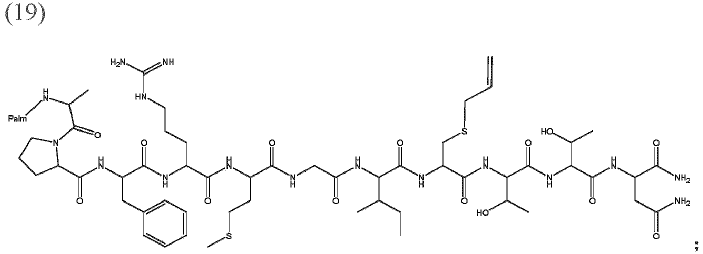 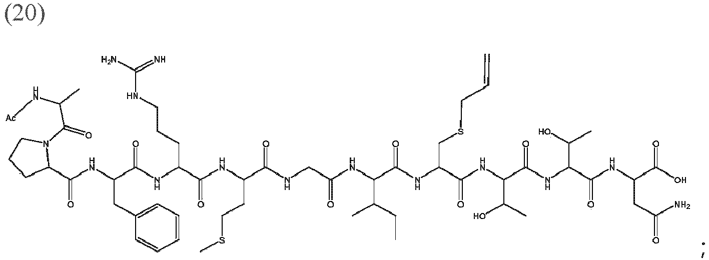 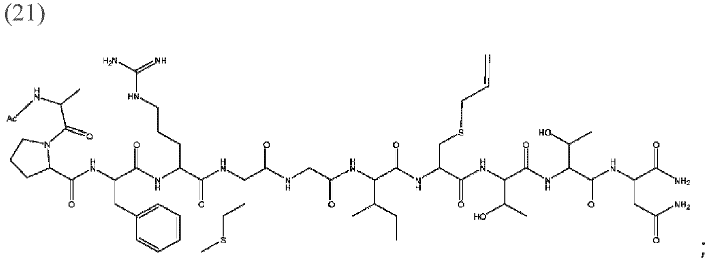 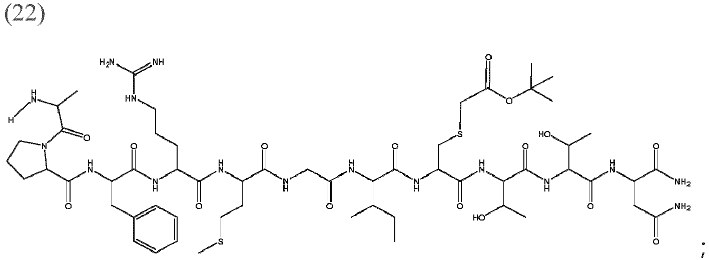 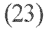 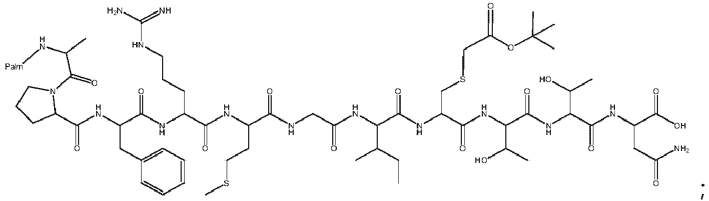 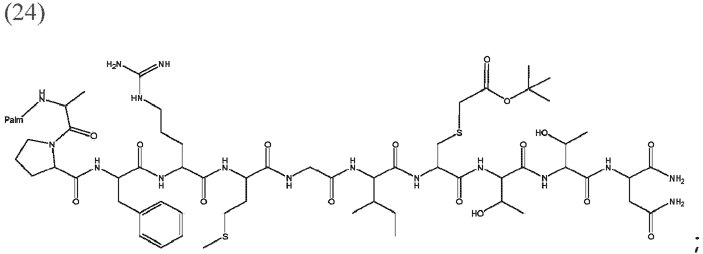 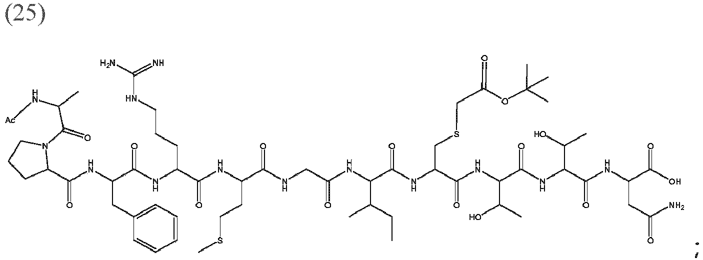 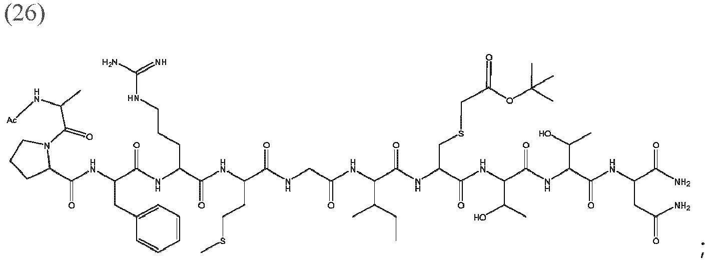 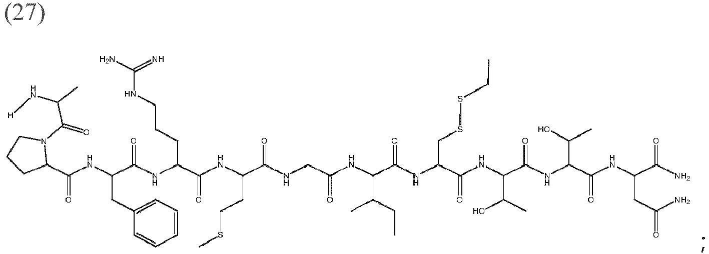 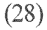 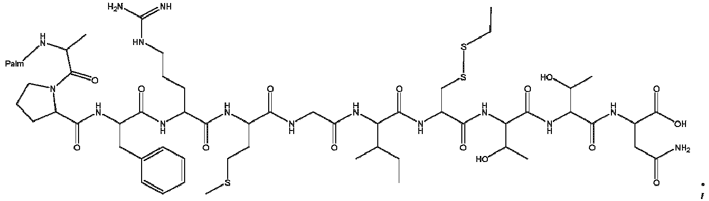 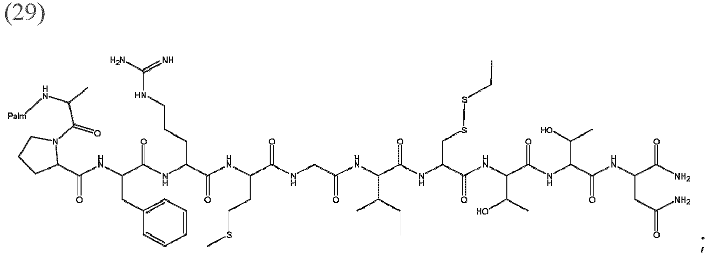 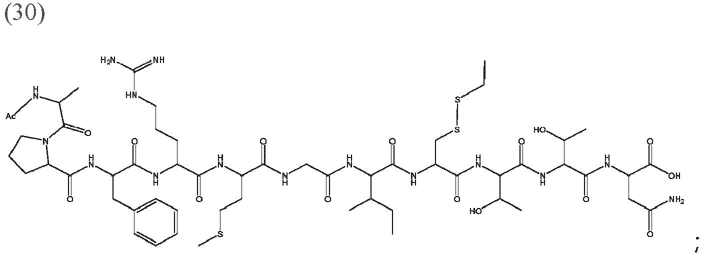 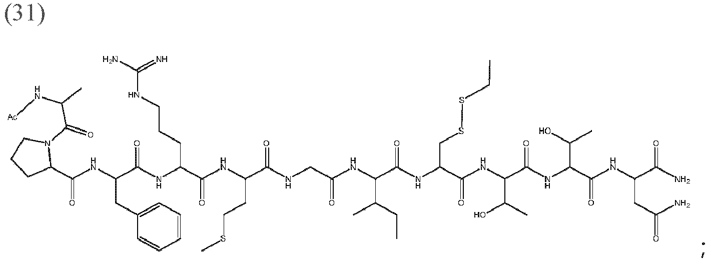 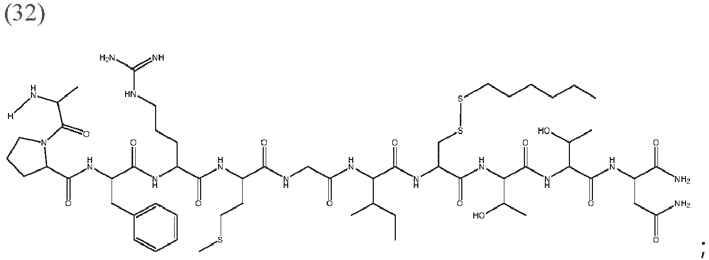 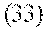 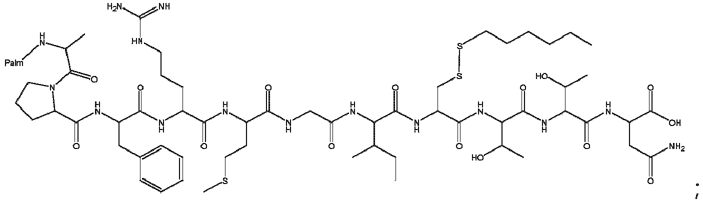 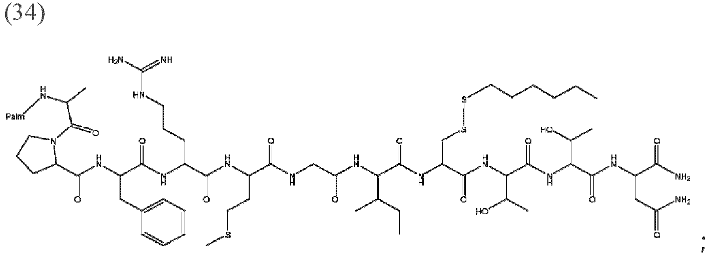 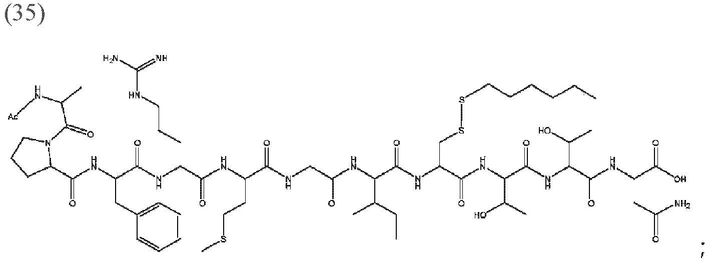 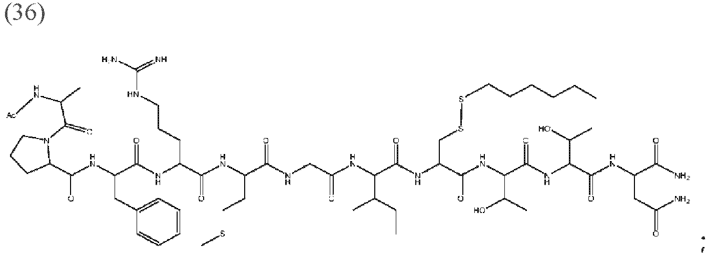 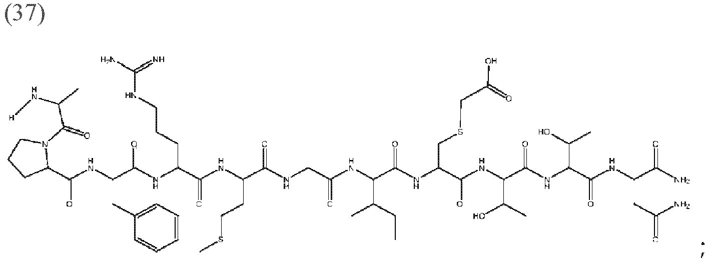 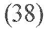 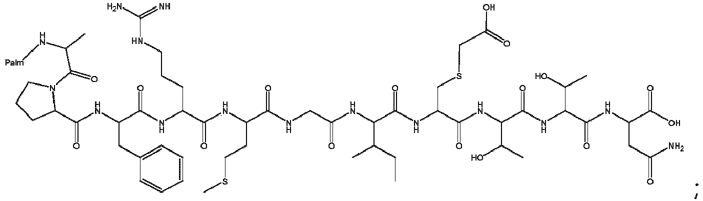 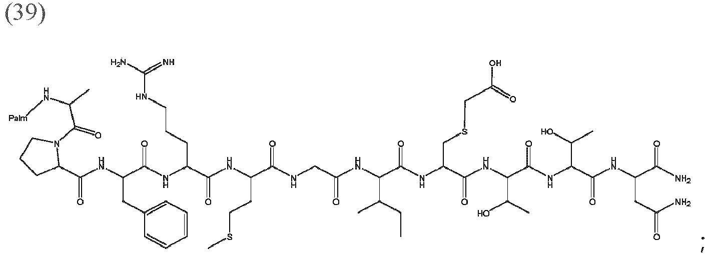 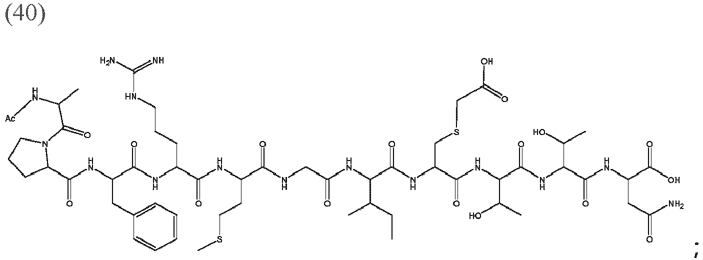 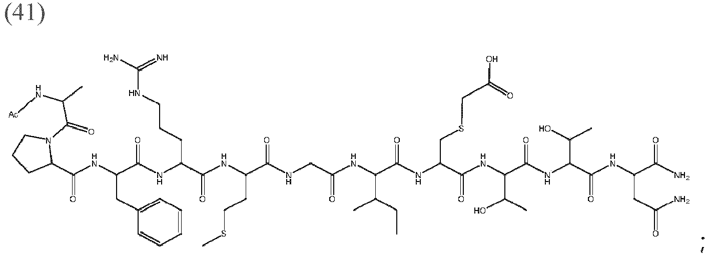
(42) H-Ala-Pro-Phe-Arg-Met-Gly-Ile-Met-Thr-Thr-Asn-NH₂;
(43) Palm-Ala-Pro-Phe-Arg-Met-Gly-Ile-Met-Thr-Thr-Asn-OH;
(44) Palm-Ala-Pro-Phe-Arg-Met-Gly-Ile-Met-Thr-Thr-Asn-NH₂;
(45) Ac-Ala-Pro-Phe-Arg-Met-Gly-Ile-Met-Thr-Thr-Asn-OH;
(46) Ac-Ala-Pro-Phe-Arg-Met-Gly-Ile-Met-Thr-Thr-Asn-NH₂.

7. The peptide represented by formula (I) according to any one of claims 1-6, or a stereoisomer thereof, or a mixture of stereoisomers thereof, or a cosmetically acceptable salt thereof, or a pharmaceutically acceptable salt thereof, **characterized in that**,
the cosmetically acceptable salt or the pharmaceutically acceptable salt includes the metal salt of the peptide shown in formula (I), and the metal includes: lithium, sodium, potassium, calcium, magnesium, manganese, copper, zinc or aluminum;
optionally, the cosmetically acceptable salt or pharmaceutically acceptable salt includes a salt formed by the peptide represented by formula (I) and an organic base, and the organic base includes: ethylamine, diethylamine, ethylenediamine, ethanolamine, diethanolamine, arginine, lysine, histidine, or piperazine;
optionally, the cosmetically acceptable salt or pharmaceutically acceptable salt includes a salt formed by the peptide represented by formula (I) and an inorganic acid or an organic acid, and the organic acid includes: acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid or gluconic acid;
optionally, the inorganic acid includes: hydrochloric acid, sulfuric acid, boric acid or carbonic acid.

8. A cosmetic or pharmaceutical composition, **characterized in that**, comprising an effective amount of the peptide represented by formula (I) according to any one of claims 1-7, or a stereoisomer thereof, or a mixture of stereoisomers thereof, or a cosmetically acceptable salt thereof, or a pharmaceutically acceptable salt thereof, and at least one excipient and optionally a cosmetically or pharmaceutically acceptable adjuvant;
optionally, the adjuvant is selected from: a collagen synthesis stimulator, agents that regulates the synthesis of PGC-1α, agents that regulates the activity of PPARγ, agents that increases or decreases the triglyceride content of adipocytes, agents that stimulates or delays the adipocyte differentiation, lipolytic agents or lipolysis stimulating agents, lipotropic agents, adipogenic agents, inhibitors of acetylcholine receptor aggregation, agents that inhibit muscle contraction, anticholinergic agents, elastase inhibitors, matrix metalloproteinases inhibitors, melanin synthesis stimulators or inhibitors, whitening or depigmenting agents, hyperpigmentation agents, self-tanning agents, antiaging agents, NO-synthase inhibitors, 5α-reductase inhibitors, lysyl hydroxylation enzyme and/or prolyl hydroxylase inhibitors, antioxidants, free radical scavengers and/or anti-air pollution agents, active carbonyl species scavengers, anti-glycation agents, antihistamines, antiviral agents, antiparasitic agents, emulsifiers, emollients, organic solvents, liquid propellants, skin conditioners, humectants, moisture retaining substances, alpha hydroxy acids, beta hydroxy acids, moisturizers, epidermal hydrolytic enzymes, vitamins, amino acids, proteins, pigments or colorants, dyes, biopolymers, gelling polymers, thickeners, surfactants, softeners, adhesives, preservatives, anti-wrinkle agents, agents that reduce or treat bags under the eyes, callus removers, exfoliating agents, keratolytic agents, antimicrobial agents, antifungal agents, fungicides, bactericides, bacteriostatic agents, agents that stimulate the synthesis of dermal or epidermal macromolecules and/or are capable of inhibiting or preventing their degradation, agents that stimulate elastin synthesis, agents that stimulate decorin synthesis, agents that stimulate laminin synthesis, agents that stimulate defensin synthesis, agents that stimulate chaperone protein synthesis, agents that stimulate cAMP synthesis, heat shock proteins, agents that stimulate the synthesis of HSP70, agents that stimulate the synthesis of heat shock proteins, agents that stimulate the synthesis of hyaluronic acid, agents that stimulate the synthesis of tibronectin, agents that stimulate the synthesis of sirtuin, agents that stimulate the synthesis of lipids and stratum corneum components, ceramide, fatty acid, agents for inhibiting collagen degradation, agents for inhibiting elastin degradation, agents for inhibiting serine protease, agents for stimulating fibroblast proliferation, agents for stimulating keratinocyte proliferation, agents for stimulating adipocyte proliferation, agents that stimulate melanocyte proliferation, agents that stimulate keratinocyte differentiation, agents that inhibit acetylcholinesterase, skin relaxants, agents that stimulate glycosaminoglycan synthesis, antihyperkeratosis agents, acne dissolving agents, antipsoriatic agents, anti-dermatitis agents, anti-eczema agents, DNA repair agents, DNA protection agents, stabilizers, antipruritic agents, agents for the treatment and/or care of sensitive skin, curing agents, firming agents, restructuring agents, anti-stretch mark agents, adhesives, agents that regulate sebum production, antiperspirants, agents that stimulate healing, agents that assist healing, agents that stimulate re-epithelialization, agents that assist re-epithelialization, cytokines, growth factors, sedatives, anti-inflammatory agents, anesthetic agents, agents acting on capillary circulation and/or microcirculation, agents stimulating angiogenesis, agents inhibiting vascular permeability, venous tense agents, agents acting on cell metabolism, agents for improving dermal-epidermal junction agents, agents that induce hair growth, agents that inhibit or delay hair growth, fragrances, chelating agents, plant extracts, essential oils, marine extracts, agents derived from biological fermentation processes, inorganic salts, cell extracts, sunscreens, and organic or inorganic light effective against A and/or B UV rays preservatives or mixtures thereof.

9. Cosmetic or pharmaceutical composition according to claim 8, **characterized in that** the formulation of said cosmetic or pharmaceutical composition is selected from creams, oils, milks, balms, foams, lotions, gels, liniments, sera, soaps, shampoos, conditioners, serums, ointments, mousses, pomades, powders, bars, pencils, sprays, aerosols, capsules, tablets, granules, chewing gums, solutions, suspensions, emulsions, syrups, elixirs, polysaccharide films, jellies or gelatins; optionally, the capsules include: soft capsules, hard capsules, optionally gelatin capsules;
optionally, the tablets include: sugar-coated tablets.

10. A cosmetically or pharmaceutically acceptable delivery system or sustained release system, **characterized in that** it comprises an effective amount of the peptide represented by formula (I) according to any one of claims 1-7, or a stereoisomer thereof, or a mixture of stereoisomers thereof, or a cosmetically acceptable salt thereof, or a pharmaceutically acceptable salt thereof, or a cosmetic or pharmaceutical composition according to claim 8 or 9;
the cosmetically or pharmaceutically acceptable delivery system or sustained release system is selected from: liposomes, oleosomes, non-ionic surfactant liposome vesicles, ethosomes, millicapsules, microcapsules, nanocapsules, nanostructured lipid carriers, sponges, cyclodextrins, lipid vesicles, micelles, millispheres, microspheres, nanospheres, lipospheres, microemulsions, nanoemulsions, milliparticles, microparticles or nanoparticles; optionally liposomes or microemulsions, optionally water-in-oil microemulsions with an internal structure of reverse micelles.

11. A cosmetically or pharmaceutically acceptable solid organic polymer or solid inorganic support, **characterized in that** it comprises an effective amount of the peptide shown in formula (I) according to any one of claims 1-7, or a stereoisomer thereof, or a mixture of stereoisomers thereof, or a cosmetically acceptable salt thereof, or a pharmaceutically acceptable salt thereof, or a cosmetic or pharmaceutical composition according to in claim 8 or 9;
the cosmetically or pharmaceutically acceptable solid organic polymer or solid inorganic support is selected from talc, bentonite, silica, starch or maltodextrin.

12. A cosmetic product, **characterized in that** it comprises an effective amount of the peptide represented by formula (I) according to any one of claims 1-7, or a stereoisomer thereof, or a mixture of stereoisomers thereof, or a cosmetically acceptable salt thereof, or a pharmaceutically acceptable salt thereof, or the cosmetic or pharmaceutical composition according to claim 8 or 9; optionally, the cosmetic product is selected from: concealers, foundations, makeup removers, makeup remover milks, eye shadows, lip sticks, lip glosses, lip balms or lip powders.

13. A fabric, nonwoven or medical device, **characterized in that** it contains an effective amount of the peptide represented by formula (I) according to any one of claims 1-7, or a stereoisomer thereof, or a mixture of stereoisomers thereof, or a cosmetically acceptable salt thereof, or a pharmaceutically acceptable salt thereof, or a cosmetic or pharmaceutical composition according to claim 8 or 9;
optionally, the fabric, nonwoven or medical device is selected from: bandages, gauzes, t-shirts, socks, pantyhoses, underwears, girdles, gloves, diapers, sanitary napkins, dressings, bedspreads, wipes, adhesive patches, non-adherent patches, occlusive patches, microelectronic patches or masks.

14. Use of the peptide represented by formula (I) according to any one of claims 1-7, or a stereoisomer thereof, or a mixture of stereoisomers thereof, or a cosmetically acceptable salt thereof, or a pharmaceutically acceptable salt thereof, or the cosmetic or pharmaceutical composition according to claim 8 or 9, or the cosmetically or pharmaceutically acceptable delivery system or sustained release system according to claim 10, or the cosmetically or pharmaceutically acceptable solid organic polymer or solid inorganic support according to claim 11, or the cosmetic product according to claim 12, or the fabric, nonwoven or medical device according to claim 13 in the preparation of cosmetic composition or pharmaceutical composition for the treatment or care of skin or mucosa.

15. Use of the peptide represented by formula (I) according to any one of claims 1-7, or a stereoisomer thereof, or a mixture of stereoisomers thereof, or a cosmetically acceptable salt thereof, or a pharmaceutically acceptable salt thereof, or the cosmetic or pharmaceutical composition according to claim 8 or 9, or the cosmetically or pharmaceutically acceptable delivery system or sustained release system according to claim 10, or the cosmetically or pharmaceutically acceptable solid organic polymer or solid inorganic support according to claim 11, or the cosmetic product according to claim 12, or the fabric, nonwoven or medical device according to claim 13 in the preparation of cosmetic composition or pharmaceutical composition for treating or nursing body surface wounds, burns, skin ulcers, reducing scar generation, or accelerating scar repair.

16. Use of the peptide represented by formula (I) according to any one of claims 1-7, or a stereoisomer thereof, or a mixture of stereoisomers thereof, or a cosmetically acceptable salt thereof , or a pharmaceutically acceptable salt thereof, or the cosmetic or pharmaceutical composition according to claim 8 or 9, or the cosmetically or pharmaceutically acceptable delivery system or sustained release system according to claim 10, or the cosmetically or pharmaceutically acceptable solid organic polymer or solid inorganic support according to claim 11, or the cosmetic product according to claim 12, or the fabric, nonwoven or medical device according to claim 13 in the preparation of cosmetic composition or pharmaceutical composition for promoting fibroblast proliferation, increasing collagen production, improving skin barrier function, or for re-epithelializing or healing the skin or mucosa.

17. Use of the peptide represented by formula (I) according to any one of claims 1-7, or a stereoisomer thereof, or a mixture of stereoisomers thereof, or a cosmetically acceptable salt thereof, or a pharmaceutically acceptable salt thereof, or the cosmetic or pharmaceutical composition according to claim 8 or 9, or the cosmetically or pharmaceutically acceptable delivery system or sustained release system according to claim 10, or the cosmetically or pharmaceutically acceptable solid organic polymer or solid inorganic support according to claim 11, or the cosmetic product according to claim 12, or the fabric, nonwoven or medical device according to claim 13 in the preparation of cosmetic composition or pharmaceutical composition for the treatment, prevention or repair of skin aging or photoaging.

18. The use according to claim 17, wherein the treatment, prevention or repair of skin aging or photoaging is to reduce, prevent or treat facial wrinkles.
